# EUROPEAN PATENT SPECIFICATION

(11) **EP 3 050 580 B1**
(45) Date of publication and mention of the grant of the patent: **19.02.2020**
(21) Application number: 14847227.7
(22) Date of filing: 24.09.2014
(51) Int. Cl.: A61L 27/00, C07K 14/78, C12N 5/077, A61L 27/38

(54) **METHOD FOR PRODUCING BIOCOMPATIBLE MACROMOLECULAR POROUS BODY, BIOCOMPATIBLE MACROMOLECULAR POROUS BODY, BIOCOMPATIBLE MACROMOLECULAR BLOCK AND CELL STRUCTURE**
VERFAHREN ZUR HERSTELLUNG EINES BIOKOMPATIBLEN MAKROMOLEKULAREN PORÖSEN KÖRPERS, BIOKOMPATIBLER MAKROMOLEKULARER PORÖSER KÖRPER, BIOKOMPATIBLER MAKROMOLEKULARER BLOCK UND ZELLSTRUKTUR
PROCÉDÉ DE PRODUCTION D'UN CORPS POREUX MACROMOLÉCULAIRE BIOCOMPATIBLE, CORPS POREUX MACROMOLÉCULAIRE BIOCOMPATIBLE, SÉQUENCE MACROMOLÉCULAIRE BIOCOMPATIBLE ET STRUCTURE CELLULAIRE

(30) Priority: 25.09.2013 JP 2013198023
(43) Date of publication of application: 03.08.2016
(73) Proprietor: FUJIFILM Corporation, Tokyo 106-8620 (JP)
(72) Inventor: NAKAMURA, Kentaro, Ashigarakami-gun Kanagawa 258-8577 (JP); IWAZAWA, Reiko, Ashigarakami-gun Kanagawa 258-8577 (JP); YAMAGUCHI, Kazuhiro, Ashigarakami-gun Kanagawa 258-8577 (JP)
(74) Representative: Hoffmann Eitle
(86) International application number: PCT/JP2014/075222
(87) International publication number: WO 2015/046216

(56) References cited:
- EP-A1- 2 564 880
- WO-A1-2011/088365
- WO-A1-2011/108517
- WO-A1-2011/108537
- WO-A1-2013/068722
- WO-A1-2013/137268
- WO-A1-2014/115732
- WO-A1-2014/133081
- WO-A2-2005/004928
- JP-A- 2006 068 080
- JP-A- 2007 222 277
- JP-A- 2012 082 245
- JP-A- 2013 517 292
- MANDAL B B ET AL: "Cell proliferation and migration in silk fibroin 3D scaffolds", BIOMATERIALS, ELSEVIER SCIENCE PUBLISHERS BV., BARKING, GB, vol. 30, no. 15, 1 May 2009 (2009-05-01), pages 2956-2965, XP026010882, ISSN: 0142-9612, DOI: 10.1016/J.BIOMATERIALS.2009.02.006 [retrieved on 2009-02-26]
- ZMORA S ET AL: "Tailoring the pore architecture in 3-D alginate scaffolds by controlling the freezing regime during fabrication", BIOMATERIALS, ELSEVIER SCIENCE PUBLISHERS BV., BARKING, GB, vol. 23, no. 20, 1 October 2002 (2002-10-01), pages 4087-4094, XP004370399, ISSN: 0142-9612, DOI: 10.1016/S0142-9612(02)00146-1
- KENTARO NAKAMURA ET AL.: 'Shinki Scaffold:RCP (Recombinant Peptide) no Tokucho to Oyo Tenkai' REGENERATIVE MEDICINE vol. 10, 2011, page 271, 2P-122, XP008180177
- REIKO IWASAWA ET AL.: 'Atsumi no Aru Ishoku-yo Saibo Kozotai (Saibo + Ashiba)-nai deno Naibu Saibo Seizon to Kekkan Yudo' REGENERATIVE MEDICINE vol. 11, 2012, pages 183, O-16 - 1

### SEQUENCE LISTING

<110> FUJIFILM Corporation
<120> A method for producing biocompatible polymer porous body, a biocompatible polymer porous body, a biocompatible polymer block, and a cell structure
<130>\201@14F00893
<160> 10
<170> PatentIn version 3.5
<210> 1
   <211> 571
   <212> PRT
   <213> Recombinant
<400> 1
   Gly Ala Pro Gly Ala Pro Gly Leu Gln Gly Ala Pro Gly Leu Gln Gly
   1               5                   10                  15
   Met Pro Gly Glu Arg Gly Ala Ala Gly Leu Pro Gly Pro Lys Gly Glu
               20                  25                  30
   Arg Gly Asp Ala Gly Pro Lys Gly Ala Asp Gly Ala Pro Gly Ala Pro
           35                  40                  45
   Gly Leu Gln Gly Met Pro Gly Glu Arg Gly Ala Ala Gly Leu Pro Gly
       50                  55                  60
   Pro Lys Gly Glu Arg Gly Asp Ala Gly Pro Lys Gly Ala Asp Gly Ala
   65                  70                  75                  80
   Pro Gly Lys Asp Gly Val Arg Gly Leu Ala Gly Pro Ile Gly Pro Pro
                   85                  90                  95
   Gly Glu Arg Gly Ala Ala Gly Leu Pro Gly Pro Lys Gly Glu Arg Gly
               100                 105                 110
   Asp Ala Gly Pro Lys Gly Ala Asp Gly Ala Pro Gly Lys Asp Gly Val
           115                 120                 125
   Arg Gly Leu Ala Gly Pro Ile Gly Pro Pro Gly Pro Ala Gly Ala Pro
       130                 135                 140
   Gly Ala Pro Gly Leu Gln Gly Met Pro Gly Glu Arg Gly Ala Ala Gly
   145                 150                 155                 160
   Leu Pro Gly Pro Lys Gly Glu Arg Gly Asp Ala Gly Pro Lys Gly Ala
                   165                 170                 175
   Asp Gly Ala Pro Gly Lys Asp Gly Val Arg Gly Leu Ala Gly Pro Pro
               180                 185                 190
   Gly Ala Pro Gly Leu Gln Gly Ala Pro Gly Leu Gln Gly Met Pro Gly
           195                 200                 205
   Glu Arg Gly Ala Ala Gly Leu Pro Gly Pro Lys Gly Glu Arg Gly Asp
       210                 215                 220
   Ala Gly Pro Lys Gly Ala Asp Gly Ala Pro Gly Ala Pro Gly Leu Gln
   225                 230                 235                 240
   Gly Met Pro Gly Glu Arg Gly Ala Ala Gly Leu Pro Gly Pro Lys Gly
                   245                 250                 255
   Glu Arg Gly Asp Ala Gly Pro Lys Gly Ala Asp Gly Ala Pro Gly Lys
               260                 265                 270
   Asp Gly Val Arg Gly Leu Ala Gly Pro Ile Gly Pro Pro Gly Glu Arg
           275                 280                 285
   Gly Ala Ala Gly Leu Pro Gly Pro Lys Gly Glu Arg Gly Asp Ala Gly
       290                 295                 300
   Pro Lys Gly Ala Asp Gly Ala Pro Gly Lys Asp Gly Val Arg Gly Leu
   305                 310                 315                 320
   Ala Gly Pro Ile Gly Pro Pro Gly Pro Ala Gly Ala Pro Gly Ala Pro
                   325                 330                 335
   Gly Leu Gln Gly Met Pro Gly Glu Arg Gly Ala Ala Gly Leu Pro Gly
               340                 345                 350
   Pro Lys Gly Glu Arg Gly Asp Ala Gly Pro Lys Gly Ala Asp Gly Ala
           355                 360                 365
   Pro Gly Lys Asp Gly Val Arg Gly Leu Ala Gly Pro Pro Gly Ala Pro
       370                 375                 380
   Gly Leu Gln Gly Ala Pro Gly Leu Gln Gly Met Pro Gly Glu Arg Gly
   385                 390                 395                 400
   Ala Ala Gly Leu Pro Gly Pro Lys Gly Glu Arg Gly Asp Ala Gly Pro
                   405                 410                 415
   Lys Gly Ala Asp Gly Ala Pro Gly Ala Pro Gly Leu Gln Gly Met Pro
               420                 425                 430
   Gly Glu Arg Gly Ala Ala Gly Leu Pro Gly Pro Lys Gly Glu Arg Gly
           435                 440                 445
   Asp Ala Gly Pro Lys Gly Ala Asp Gly Ala Pro Gly Lys Asp Gly Val
       450                 455                 460
   Arg Gly Leu Ala Gly Pro Ile Gly Pro Pro Gly Glu Arg Gly Ala Ala
   465                 470                 475                 480
   Gly Leu Pro Gly Pro Lys Gly Glu Arg Gly Asp Ala Gly Pro Lys Gly
                   485                 490                 495
   Ala Asp Gly Ala Pro Gly Lys Asp Gly Val Arg Gly Leu Ala Gly Pro
               500                 505                 510
   Ile Gly Pro Pro Gly Pro Ala Gly Ala Pro Gly Ala Pro Gly Leu Gln
           515                 520                 525
   Gly Met Pro Gly Glu Arg Gly Ala Ala Gly Leu Pro Gly Pro Lys Gly
       530                 535                 540
   Glu Arg Gly Asp Ala Gly Pro Lys Gly Ala Asp Gly Ala Pro Gly Lys
   545                 550                 555                 560
   Asp Gly Val Arg Gly Leu Ala Gly Pro Pro Gly
                   565                 570
<210> 2
   <211> 4
   <212> PRT
   <213> Artificial Sequence
<220>
   <223> Description of Artificial Sequence: Cell adhesion signal
<400> 2
   Arg Glu Asp Val
   1
<210> 3
   <211> 5
   <212> PRT
   <213> Artificial Sequence
<220>
   <223> Description of Artificial Sequence: Cell adhesion signal
<400> 3
   Tyr Ile Gly Ser Arg
   1               5
<210> 4
   <211> 5
   <212> PRT
   <213> Artificial Sequence
<220>
   <223> Description of Artificial Sequence: Cell adhesion signal
<400> 4
   Pro Asp Ser Gly Arg
   1               5
<210> 5
   <211> 7
   <212> PRT
   <213> Artificial Sequence
<220>
   <223> Description of Artificial Sequence: Cell adhesion signal
<400> 5
   Arg Tyr Val Val Leu Pro Arg
   1 5
<210> 6
   <211> 6
   <212> PRT
   <213> Artificial Sequence
<220>
   <223> Description of Artificial Sequence: Cell adhesion signal
<400> 6
   Leu Gly Thr Ile Pro Gly
   1 5
<210> 7
   <211> 10
   <212> PRT
   <213> Artificial Sequence
<220>
   <223> Description of Artificial Sequence: Cell adhesion signal
<400> 7
   Arg Asn Ile Ala Glu Ile Ile Lys Asp Ile
   1 5 10
<210> 8
   <211> 5
   <212> PRT
   <213> Artificial Sequence
<220>
   <223> Description of Artificial Sequence: Cell adhesion signal
<400> 8
   Ile Lys Val Ala Val
   1 5
<210> 9
   <211> 4
   <212> PRT
   <213> Artificial Sequence
<220>
   <223> Description of Artificial Sequence: Cell adhesion signal
<400> 9
   Asp Gly Glu Ala
   1
<210> 10
   <211> 4
   <212> PRT
   <213> Artificial Sequence
<220>
   <223> Description of Artificial Sequence: Cell adhesion signal
<400> 10
   Glu Arg Gly Asp
    1

## Description

### BACKGROUND OF THE INVENTION

### 1. Field of the Invention

The present invention relates to a method for producing biocompatible macromolecular porous body, a biocompatible macromolecular porous body, a biocompatible macromolecular block, and a cell structure. Specifically, the present invention relates to a biocompatible macromolecular porous body which can produce a cell structure showing a high number of cells and a high number of blood vessels, and a method for producing the same; and a biocompatible macromolecular block and a cell structure which can be obtained from the biocompatible macromolecular porous body.

### 2. Description of the Related Art

Currently, regenerative medicine, which regenerates living body tissues and organs having functional disorders or dysfunction, is put into practical use. The regenerative medicine is new medical technology creating a form or a function of a living body tissue that cannot be recovered with only natural healing ability possessed by a living body, which is the same as that of an original tissue, again, using three factors including a cell, a scaffold, and a growth factor. In recent years, treatment using cells is gradually realized. Examples thereof include cartilage treatment using autologous chondrocytes, and cultured epidermis using autologous cells; bone regeneration treatment using mesenchymal stem cells; myocardial cell sheet treatment using myoblasts; cornea regeneration treatment using corneal epithelial sheets; and nerve regeneration treatment. These kinds of new treatment are different from alternative medicine (for example, a bone prosthetic material or hyaluronic acid injection) using an artifact in the related art, and repair and regenerate biological tissues, thereby obtaining a high treatment effect. Actually, products such as cultured epidermis or cultured cartilage using autologous cells have been available on the market.

For example, when regenerating the myocardium using cell sheets, it is considered that a multilayer structure of the cell sheets is required in order to regenerate a tissue with thickness.

Furthermore, in WO2011/108517A and US2013/071441A, there is a disclosure of a three-dimensional cell structure which is produced by three-dimensionally arranging a cell and a biocompatible macromolecular block in a mosaic shape. In this three-dimensional cell structure, it is possible to deliver nutrients to the inside of the three-dimensional cell structure from the outside. The three-dimensional cell structure has a sufficient thickness, and cells evenly exist in the structure. In addition, in Example of WO2011/108517A, high cell survival activity is verified using a macromolecular block formed of a recombinant gelatin material or a natural gelatin material, and in US2013/071441A, there is a disclosure that it is possible to form a blood vessel in a transplantation site after transplanting a three-dimensional cell structure.

B. MANDAL et al. "Cell proliferation and migration in silk fibroin 3D scaffolds", BIOMATERIALS, 30 (15), 01-05-2009, 2956-2965, XP026010882; S. ZMORA et al. "Tailoring the pore architecture in 3-D alginate scaffolds by controlling the freezing regime during fabrication", BIOMATERIALS, 23 (20), 01-10-2002, 4087-4094, XP004370399; and WO2013068722 disclose, that in the preparation of lyophilised porous biomaterial scaffolds slow cooling leads to isotropic/uniform pore structures.

### SUMMARY OF THE INVENTION

The three-dimensional cell structure disclosed in WO2011/108517A and US2013/071441A shows high cell survival activity and high angiogenic ability. However, a biocompatible macromolecular block and a cell structure which show higher cell survival activity and higher angiogenic ability are desired.

Therefore, an object of the present invention to be solved is to provide a biocompatible macromolecular porous body, which enables provision of a cell structure showing a high number of cells and a high number of blood vessels, and a method for producing the same. Furthermore, another object of the present invention is to provide a biocompatible macromolecular block and a cell structure which can be obtained from the biocompatible macromolecular porous body.

The present inventors have conducted intensive studies in order to solve the above-described problems, and as a result, they have found that the variation in sizes of porous pores is small, that is, the structural evenness of a porous body is high, in the biocompatible macromolecular porous body which is produced through a step (a) of cooling a solution of biocompatible macromolecules to be in an unfrozen state, the difference between a temperature of a portion at the highest liquid temperature within the solution and a temperature of a portion at the lowest liquid temperature within the solution being lower than or equal to 2.5°C and the temperature of the portion at the highest liquid temperature within the solution being lower than or equal to a melting point of a solvent; a step (b) of freezing the solution of biocompatible macromolecules obtained in the step (a); and a step (c) of freeze-drying the frozen biocompatible macromolecules obtained in the step (b). Furthermore, the present inventors have found that a cell structure produced using the biocompatible macromolecular porous body obtained in this manner shows a high number of cells and a high number of blood vessels. The present invention has been completed based on these findings.

That is, according to the present invention, there is provided a method for producing a biocompatible macromolecular porous body which includes:
a step (a) of cooling a solution of biocompatible macromolecules to be in an unfrozen state, the difference between a temperature of a portion at the highest liquid temperature within the solution and a temperature of a portion at the lowest liquid temperature within the solution being lower than or equal to 2.5°C and the temperature of the portion at the highest liquid temperature within the solution being lower than or equal to a melting point of a solvent;
a step (b) of freezing the solution of biocompatible macromolecules obtained in the step (a); and
a step (c) of freeze-drying the frozen biocompatible macromolecules obtained in the step (b),
wherein the biocompatible macromolecules are gelatin, collagen, elastin, fibronectin, laminin, tenascin, fibrin, fibroin, entactin, thrombospondin, polylactic acid, polyglycolic acid, lactic acid-glycolic acid copolymers, hyaluronic acid, glycosaminoglycans, proteoglycans, chondroitin, cellulose, agarose, carboxymethyl cellulose, chitin, or chitosan.

It is preferable that, in the step (a), the difference between a temperature of a portion at the highest liquid temperature within the solution and a temperature of a portion at the lowest liquid temperature within the solution immediately before generation of solidification heat is lower than or equal to 2.5°C.

It is preferable that, in the step (a), the temperature of the portion at the lowest liquid temperature within the solution is lower than or equal to a melting point of the solvent -5°C.

According to the present invention, there is provided a biocompatible macromolecular porous body produced through the method for producing a biocompatible macromolecular porous body of the present invention.

According to the present invention, there is further provided a biocompatible macromolecular porous body, in which, with respect to a two-dimensional Fourier transformation image which is obtained by performing two-dimensional Fourier transformation on 1.5 mm square field of view of an image of a cross-sectional structure of the biocompatible macromolecular porous body, in a case where a line profile, in which brightness values in the vicinity of an x-axis coordinate with respect to one tenth pixel width of the pixel size of the image from a lower end of an y-axis of the two-dimensional Fourier transformation image are plotted on the y-axis by being averaged, is created, there is no peak at a region of half of the maximum value of x on the line profile, wherein the biocompatible macromolecules are gelatin, collagen, elastin, fibronectin, laminin, tenascin, fibrin, fibroin, entactin, thrombospondin, polylactic acid, polyglycolic acid, lactic acid-glycolic acid copolymers, hyaluronic acid, glycosaminoglycans, proteoglycans, chondroitin, cellulose, agarose, carboxymethyl cellulose, chitin, or chitosan. It is preferable that this biocompatible macromolecular porous body is produced through the method for producing a biocompatible macromolecular porous body of the present invention.

It is preferable that, in a case where a variation value of a base of the line profile is set to σ, a case, in which a peak of greater than or equal to 2.0 σ is not detected at a region of half of the maximum value of x on the line profile, is regarded as having no peak.

The biocompatible macromolecules are gelatin, collagen, elastin, fibronectin, laminin, tenascin, fibrin, fibroin, entactin, thrombospondin, polylactic acid, polyglycolic acid, lactic acid-glycolic acid copolymers, hyaluronic acid, glycosaminoglycans, proteoglycans, chondroitin, cellulose, agarose, carboxymethyl cellulose, chitin, or chitosan.

It is preferable that the biocompatible macromolecules are recombinant gelatin.

It is preferable that the recombinant gelatin is represented by

Formula: A-[(Gly-X-Y)n]m-B

(where A represents an arbitrary amino acid or an amino acid sequence, B represents an arbitrary amino acid or an amino acid sequence, n pieces of X each independently represent any amino acid, n pieces of Y each independently represent any amino acid, n represents an integer of 3 to 100, and m represents an integer of 2 to 10. n pieces of Gly-X-Y may be the same as or different from each other).

It is preferable that the recombinant gelatin is any of
(a) protein having an amino acid sequence described in SEQ ID No: 1;
(b) protein which has an amino acid sequence in which one or a plurality of amino acids are deleted, substituted, or added in the amino acid sequence described in SEQ ID No: 1, and has biocompatibility; or
(c) protein which has an amino acid sequence having 80% or higher homology to the amino acid sequence described in SEQ ID No: 1, and has biocompatibility.

It is preferable that the recombinant gelatin is protein which has an amino acid sequence having 90% or higher homology to an amino acid sequence described in SEQ ID No: 1, and has biocompatibility.

It is preferable that the recombinant gelatin is protein which has an amino acid sequence having 95% or higher homology to an amino acid sequence described in SEQ ID No: 1, and has biocompatibility.

It is preferable that the biocompatible macromolecules in the porous body are cross-linked using heat, ultraviolet rays, or enzyme.

According to the present invention, there is further provided a biocompatible macromolecular block which is obtained by grinding the biocompatible macromolecular porous body according to the present invention.

According to the present invention, there is further provided a cell structure containing: the biocompatible macromolecular block according to the present invention; and at least one kind of cell, in which a plurality of the biocompatible macromolecular blocks are arranged in a gap between a plurality of cells.

It is preferable that the size of one biocompatible macromolecular block is 20 µm to 200 µm.

It is preferable that the size of one biocompatible macromolecular block is 50 µm to 120 µm.

It is preferable that the thickness or the diameter of the cell structure of the present invention is 400 µm to 3 cm.

It is preferable that the cell structure of the present invention contains 0.0000001 µg to 1 µg of a biocompatible macromolecular block per cell.

It is preferable that the cell structure of the present invention contains an angiogenic factor.

It is preferable that the cells are cells selected from the group consisting of pluripotent cells, somatic stem cells, precursor cells, and mature cells.

It is preferable that the cells are only non-vascular cells.

It is preferable that the cells contain both of the non-vascular cells and vascular cells.

It is preferable that the cell structure has a region in which the area of the vascular cells in the central portion is larger than that of the vascular cells in the peripheral portion, in the cell structure.

It is preferable that the cell structure has a region in which the proportion of the vascular cells in the central portion is 60% to 100% with respect to the total area of the vascular cells.

It is preferable that the cell structure has a region in which the density of the vascular cells in the central portion of the cell structure is greater than or equal to 1.0 x 10⁻⁴ cells/µm³.

It is preferable that blood vessels are formed inside the cell structure.

The cell structure, which is produced using the biocompatible macromolecular porous body produced through the method for producing a biocompatible macromolecular porous body according to the present invention, can show a high number of cells and a high number of blood vessels in the cell structure. According to the cell structure provided by the present invention, it is possible to perform more effective cell transplantation treatment.

### BRIEF DESCRIPTION OF THE DRAWINGS

Fig. 1 shows a liquid temperature profile of a test described in "A".
Fig. 2 shows a liquid temperature profile of a test described in "B".

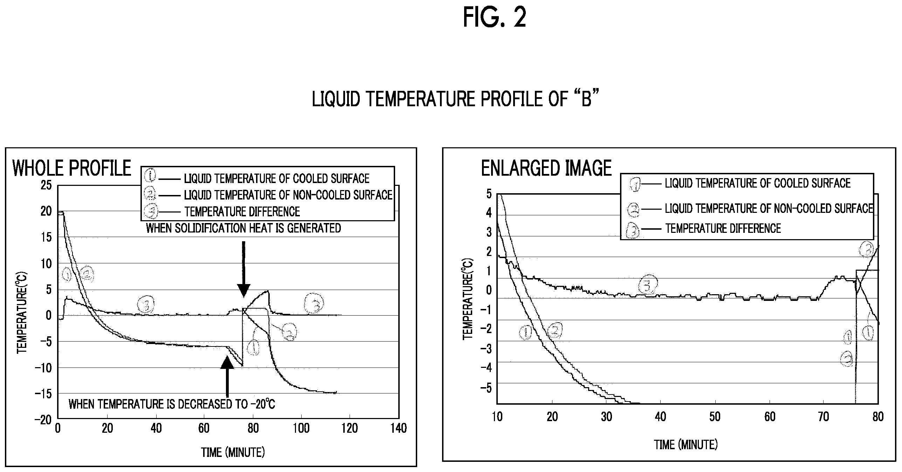

Fig. 2 shows a liquid temperature profile of a test described in "C".

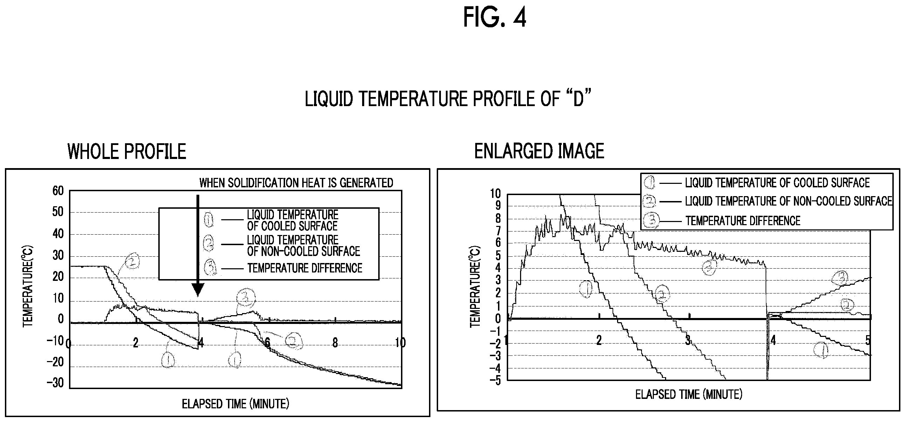

Fig. 4 shows a liquid temperature profile of a test described in "D".

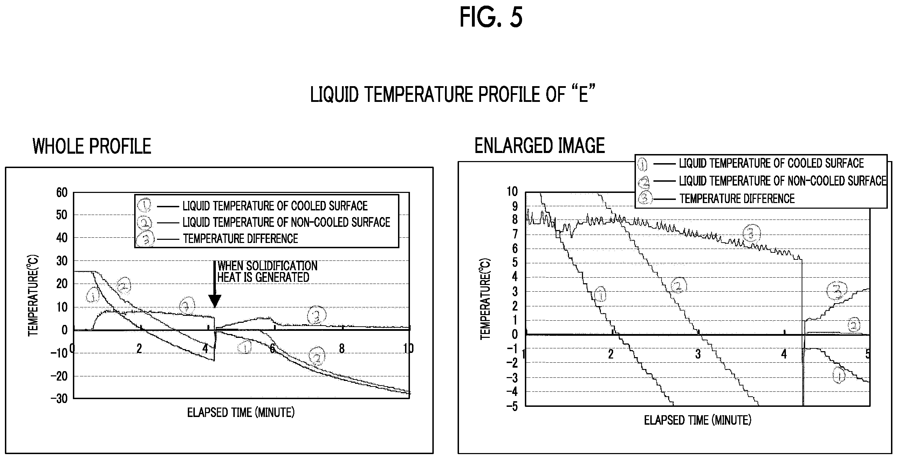

Fig. 5 shows a liquid temperature profile of a test described in "E".

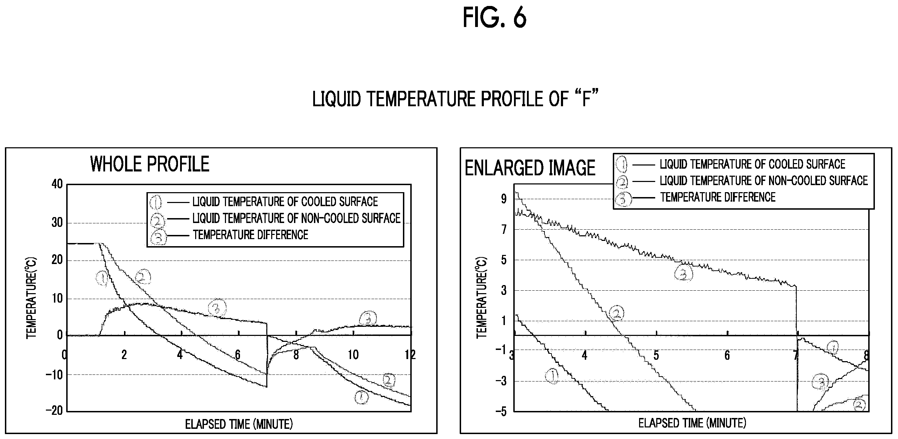

Fig. 6 shows a liquid temperature profile of a test described in "F".

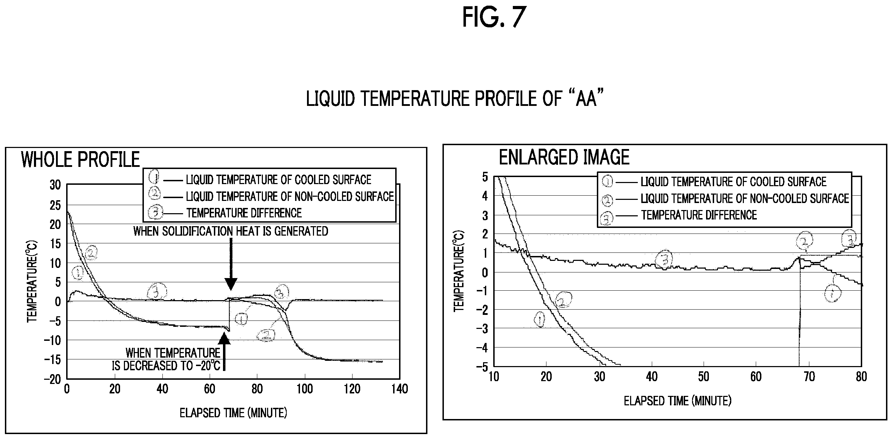

Fig. 7 shows a liquid temperature profile of a test described in "AA".

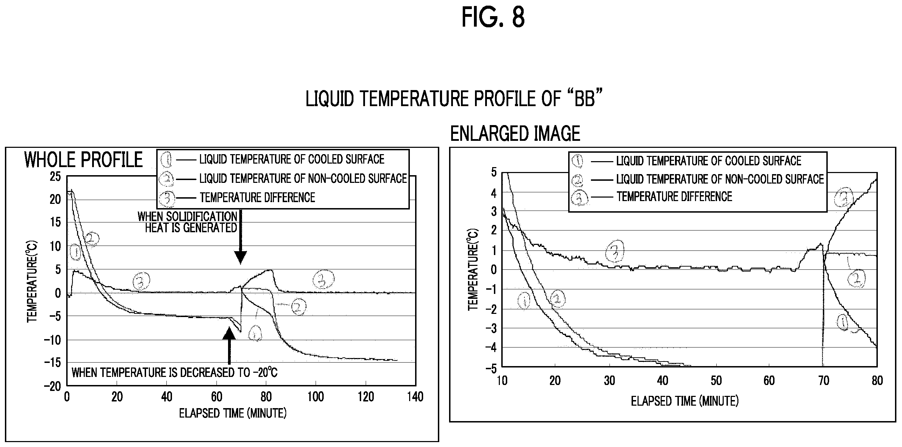

Fig. 8 shows a liquid temperature profile of a test described in "BB".

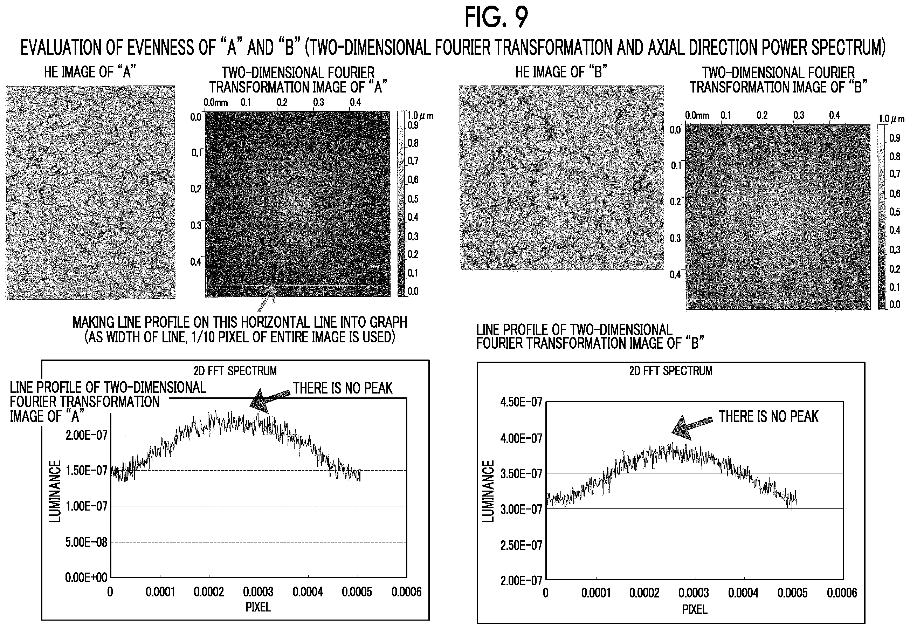

Fig. 9 shows an evaluation (two-dimensional Fourier transformation and axis direction power spectrum) of evenness of CBE3 porous body obtained in "A" and "B".

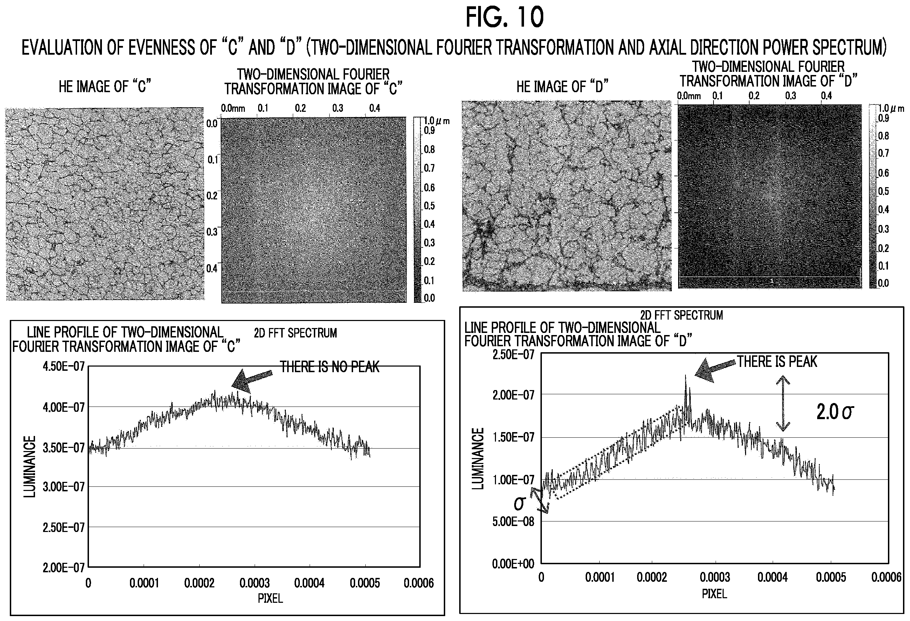

Fig. 10 shows an evaluation (two-dimensional Fourier transform and axis direction power spectrum) of evenness of CBE3 porous body obtained in "C" and "D".

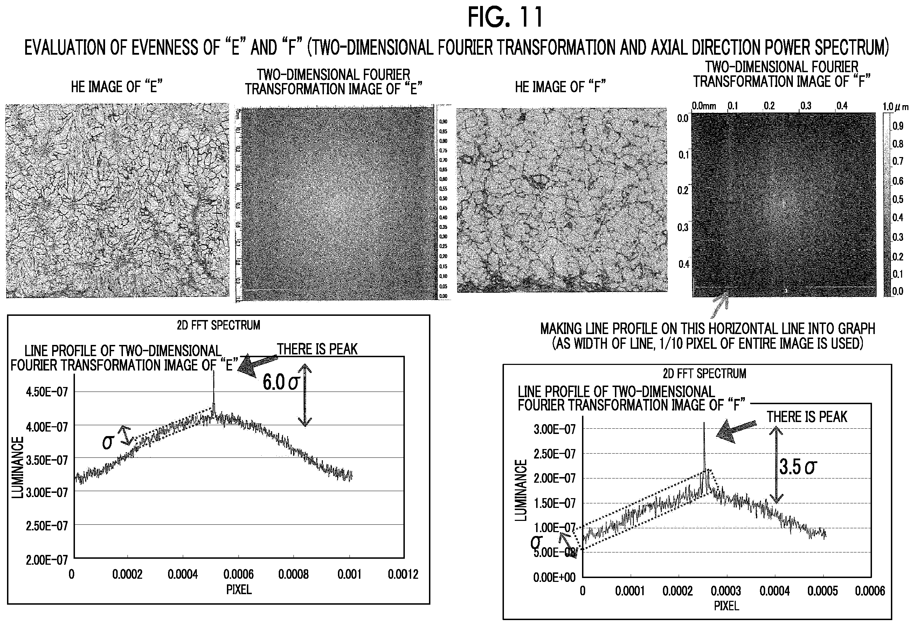

Fig. 11 shows an evaluation (two-dimensional Fourier transform and axis direction power spectrum) of evenness of CBE3 porous body obtained in "E" and "F".

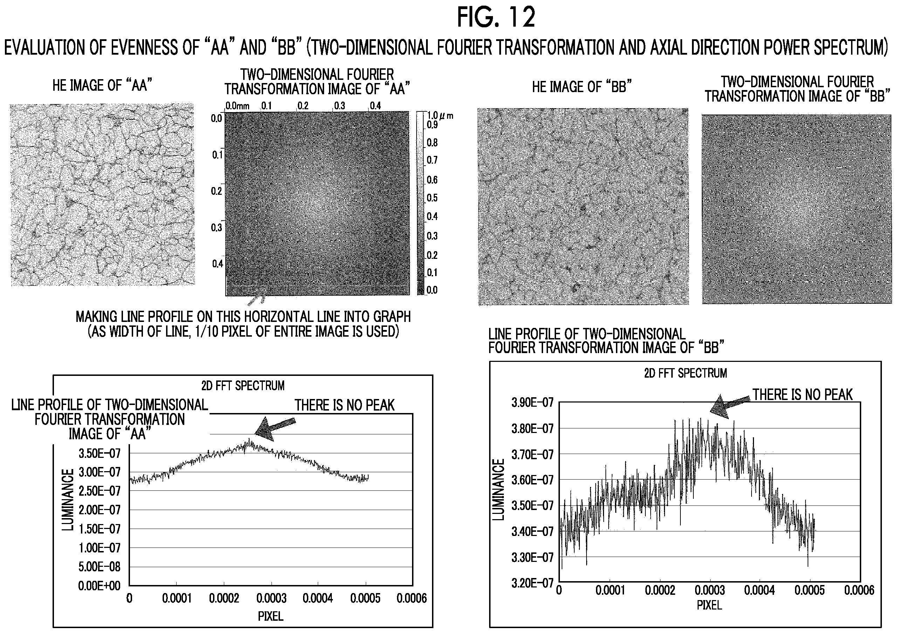

Fig. 12 shows an evaluation (two-dimensional Fourier transform and axis direction power spectrum) of evenness of CBE3 porous body obtained in "AA" and "BB".

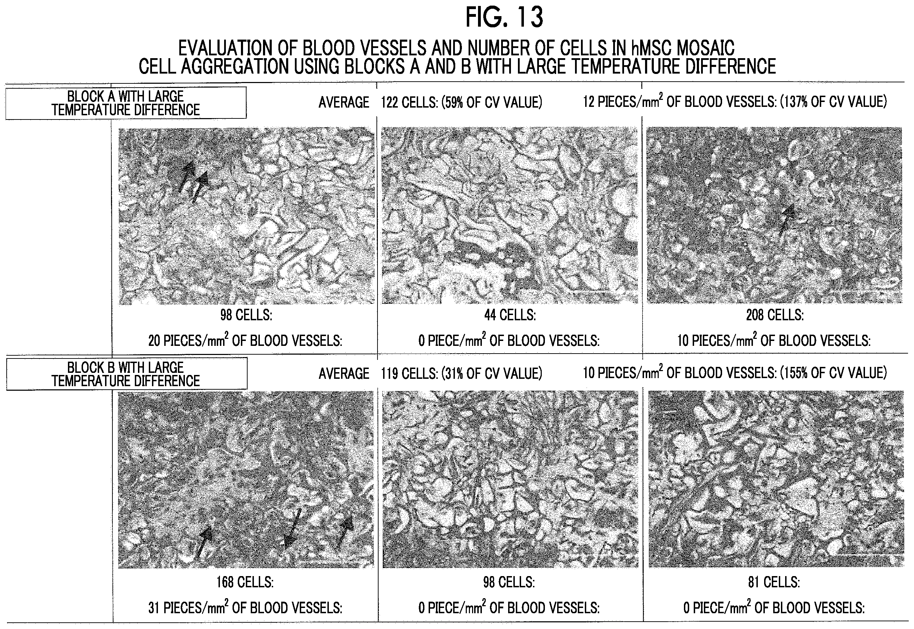

Fig. 13 shows an evaluation of blood vessels and the number of cells in an hMSC mosaic cell aggregation using blocks A and B with a large temperature difference.

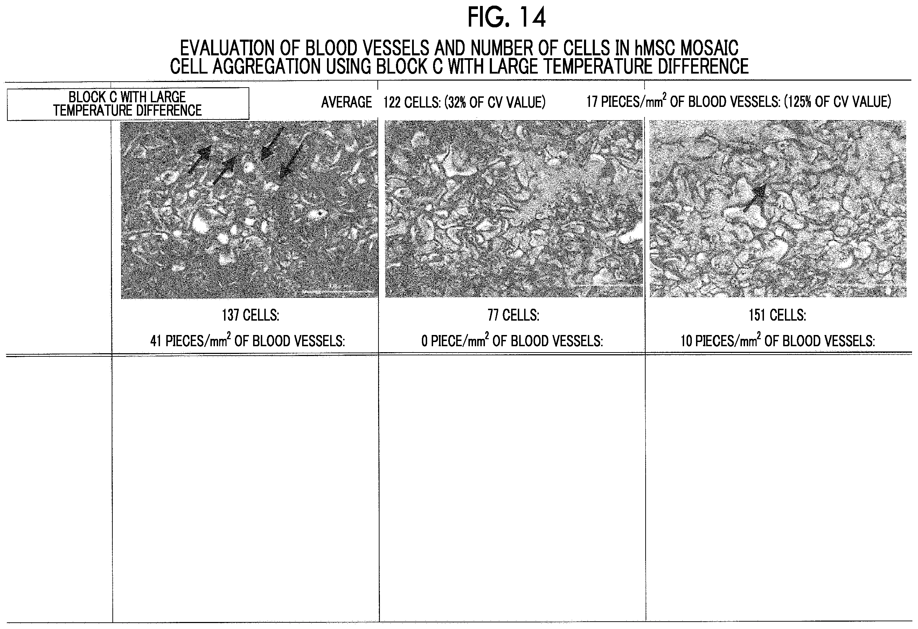

Fig. 14 shows an evaluation of blood vessels and the number of cells in an hMSC mosaic cell aggregation using a block C with a large temperature difference.

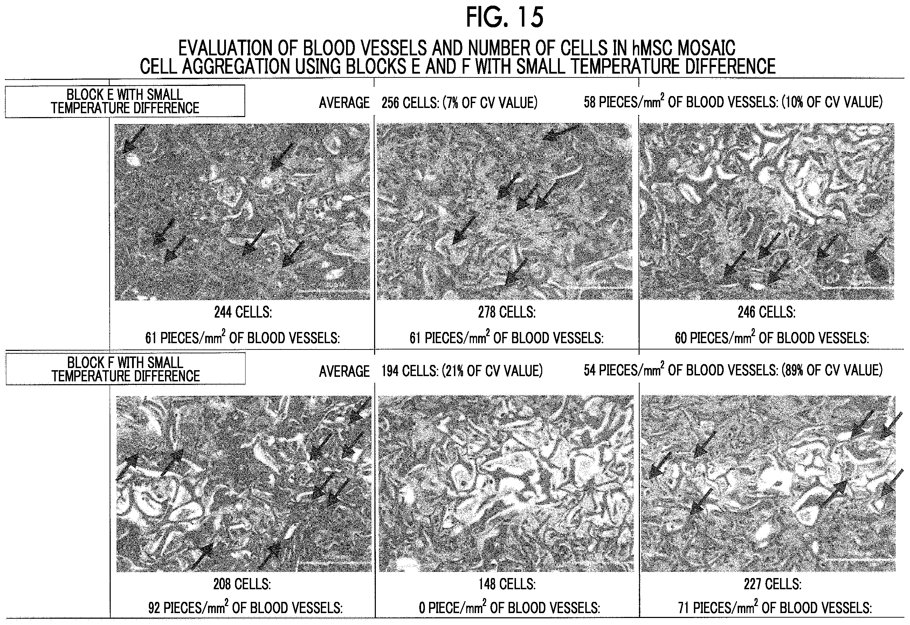

Fig. 15 shows an evaluation of blood vessels and the number of cells in an hMSC mosaic cell aggregation using blocks E and F with a small temperature difference.

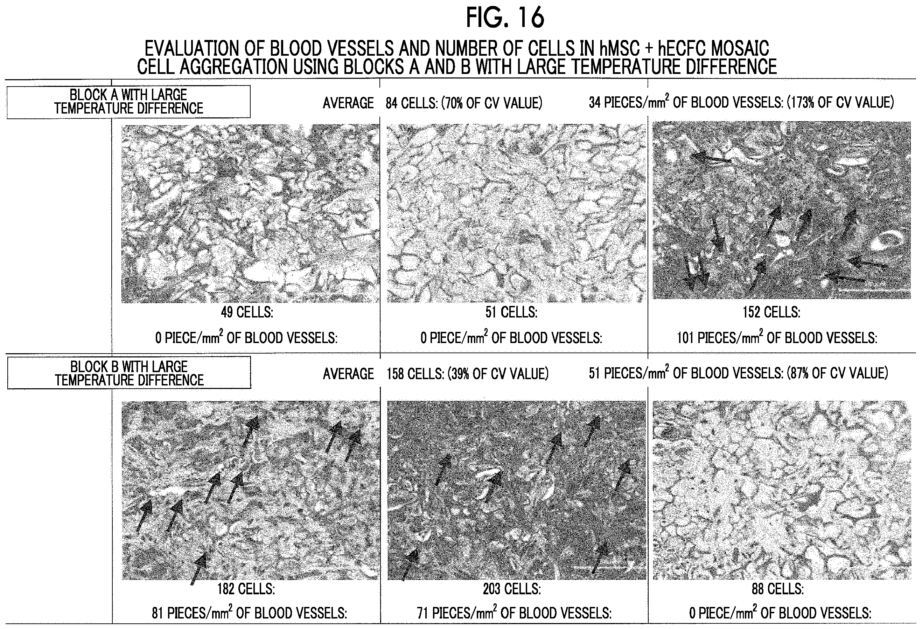

Fig. 16 shows an evaluation of blood vessels and the number of cells in an hMSC + hECFC mosaic cell aggregation using blocks A and B with a large temperature difference.

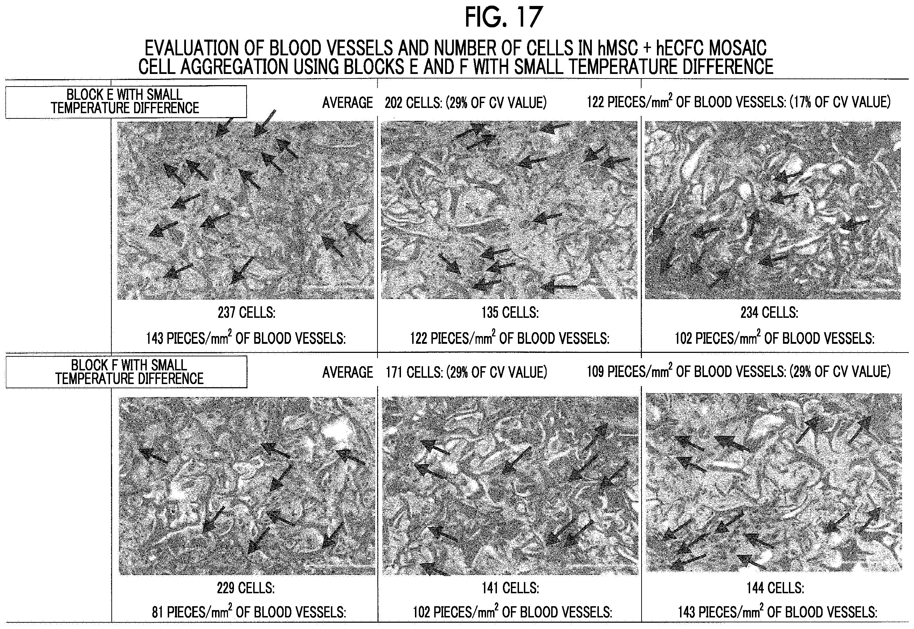

Fig. 17 shows an evaluation of blood vessels and the number of cells in an hMSC + hECFC mosaic cell aggregation using blocks E and F with a small temperature difference.

### DESCRIPTION OF THE PREFERRED EMBODIMENTS

Hereinafter, an embodiment of the present invention will be described in detail.

### (1) Method for Producing Biocompatible Macromolecular Porous Body

A method for producing a biocompatible macromolecular porous body of the present invention includes:
a process (a) of cooling a solution of biocompatible macromolecules to be in an unfrozen state, the difference between a temperature of a portion at the highest liquid temperature within the solution and a temperature of a portion at the lowest liquid temperature within the solution being lower than or equal to 2.5°C and the temperature of the portion at the highest liquid temperature within the solution being lower than or equal to a melting point of a solvent;
a process (b) of freezing the solution of biocompatible macromolecules obtained in the process (a); and
a process (c) of freeze-drying the frozen biocompatible macromolecules obtained in the process (b), wherein the biocompatible macromolecules are gelatin, collagen, elastin, fibronectin, laminin, tenascin, fibrin, fibroin, entactin, thrombospondin, polylactic acid, polyglycolic acid, lactic acid-glycolic acid copolymers, hyaluronic acid, glycosaminoglycans, proteoglycans, chondroitin, cellulose, agarose, carboxymethyl cellulose, chitin, or chitosan.

In the method of the present invention, when cooling a solution of biocompatible macromolecules to be in an unfrozen state, the variation in sizes of obtained porous pores becomes small by making the difference between a temperature of a portion at the highest liquid temperature and a temperature of a portion at the lowest liquid temperature within the solution be lower than or equal to 2.5 °C (preferably lower than or equal to 2.3°C and more preferably lower than or equal to 2.1 °C), that is, by making the difference in temperature small. The lower limit of the difference between a temperature of a portion at the highest liquid temperature and a temperature of a portion at the lowest liquid temperature within the solution is not particularly limited, and may be higher than or equal to 0°C. For example, the lower limit thereof may be higher than or equal to 0.1°C, higher than or equal to 0.5°C, higher than or equal to 0.8°C, or higher than or equal to 0.9°C. Accordingly, an effect is achieved in which the cell structure using the biocompatible macromolecular block produced using the porous body produced through the method of the present invention shows a high number of cells and a high number of blood vessels.

The cooling in the process (a) is preferably performed using a material (preferably Teflon (registered trademark)) having a lower thermal conductivity than that of water. The portion at the highest liquid temperature within the solution can be assumed as a portion farthest from the cooling side, and the portion at the lowest liquid temperature within the solution can be assumed as a liquid temperature on the cooling surface.

In the process (a), the difference between a temperature of a portion at the highest liquid temperature within the solution and a temperature of a portion at the lowest liquid temperature within the solution immediately before generation of solidification heat is preferably lower than or equal to 2.5°C, more preferably lower than or equal to 2.3°C, and still more preferably lower than or equal to 2.1°C. Here, the "difference in temperature immediately before the generation of solidification heat" means a difference in temperature when the difference in temperature becomes largest between one second before the generation of solidification heat and 10 seconds before the generation of solidification heat.

In the process (a), the temperature of the portion at the lowest liquid temperature within the solution is preferably lower than or equal to a melting point of the solvent -5°C, more preferably the melting point of the solvent -20°C to the melting point of the solvent -5°C, and still more preferably the melting point of the solvent -16°C to the melting point of the solvent -6°C. The solvent in the solvent melting point is a solvent of a solution of biocompatible macromolecules.

In the process (b), the solution of biocompatible macromolecules which has been obtained in the process (a) is frozen. The cooling temperature for the solution to be frozen in the process (b) is not particularly limited. Depending on the equipment for performing the cooling, the cooling temperature is preferably a temperature which is 3°C to 30°C lower than the temperature of the portion at the lowest liquid temperature within the solution, more preferably a temperature which is 5°C to 25°C lower than the temperature of the portion at the lowest liquid temperature within the solution, and still more preferably a temperature which is 10°C to 20°C lower than the temperature of the portion at the lowest liquid temperature within the solution.

In the process (c), the frozen biocompatible macromolecules which have been obtained in the process (b) are freeze-dried. The freeze-drying can be performed through a usual method. For example, it is possible to perform the freeze-drying by performing vacuum drying at a temperature lower than the melting point of the solvent and by further performing vacuum drying at room temperature (20°C).

### (2) Biocompatible Macromolecular Porous Body

According to the present invention, there is provided a biocompatible macromolecular porous body which is produced through the above-described method for producing a biocompatible macromolecular porous body.

According to the present invention, there is further provided a biocompatible macromolecular porous body, in which, with respect to a two-dimensional Fourier transformation image which is obtained by performing two-dimensional Fourier transformation on 1.5 mm square field of view of an image of a cross-sectional structure of the biocompatible macromolecular porous body, in a case where a line profile, in which brightness values in the vicinity of an x-axis coordinate with respect to one tenth pixel width of the pixel size of the image from a lower end of an y-axis of the two-dimensional Fourier transformation image are plotted on the y-axis by being averaged, is created, there is no peak at a region where x on the line profile is half of the maximum value, wherein the biocompatible macromolecules are gelatin, collagen, elastin, fibronectin, laminin, tenascin, fibrin, fibroin, entactin, thrombospondin, polylactic acid, polyglycolic acid, lactic acid-glycolic acid copolymers, hyaluronic acid, glycosaminoglycans, proteoglycans, chondroitin, cellulose, agarose, carboxymethyl cellulose, chitin, or chitosan.

Here, "there is no peak at a region where x on the line profile is half of the maximum value" preferably means a case, in which a peak of greater than or equal to 2.0 σ is not detected at a region of half of the maximum value of x on the line profile, in a case where a variation value of a base of the line profile is set to σ.

The method for obtaining the image of a cross-sectional structure of a biocompatible macromolecular porous body is not particularly limited. For example, it is possible to acquire the image thereof by producing a frozen slice and producing a Hematoxylin-Eosin (HE) dye sample. A two-dimensional Fourier transformation image is acquired by performing two-dimensional Fourier transformation on 1.5 mm square field of view in the obtained sample image. The two-dimensional Fourier transformation can be performed using, for example, image analysis software ImageJ.

In the obtained two-dimensional Fourier transformation image, in a case where uneven directional properties are confirmed in the biocompatible macromolecular porous body, a strong power spectrum is observed particularly in the axial direction. The presence and absence of the power spectrum in the axial direction can be defined by taking a line profile with respect to the lower end of the y-axis of the two-dimensional Fourier transformation image. Specifically, the line profile is created by averaging brightness values in the vicinity of an x-axis coordinate with respect to one tenth pixel width of the pixel size of the image from a lower end of an y-axis and plotting the average brightness value on the y-axis. The creation of a line profile can be performed using, for example, software "Gwyddion 2.31". As described above, the presence and absence of the power spectrum can be defined by acquiring the brightness values from one end to the other end of an image in a transverse direction of the image and by digitizing (line spectrum) the obtained brightness values. In the x-coordinate of the line profile, the left end in the two-dimensional Fourier transformation image is set to zero and the position advanced by one pixel to the right is set to 0.000001. That is, at the position advanced by 512 pixels from the left end in the two-dimensional Fourier transformation image, the x-coordinate on the fine profile becomes 0.000512.

Criteria for determination of the presence and absence of the power spectrum is not particularly limited, but for example, when a variation value of a base of the obtained line profile is set to σ, in a case where a peak of greater than or equal to 2.0 σ is not detected in a central portion (at a region where x on the line profile is half of the maximum value), it can be determined that there is no power spectrum in the axial direction, and in a case where a peak of greater than or equal to 2.0 σ is detected in a central portion (at a region where x on the line profile is half of the maximum value), it can be determined that there is a power spectrum in the axial direction.

As the "porous body" in the present invention, it is possible to use materials which have a plurality of "hollow holes with a size of 10 µm to 500 µm" on the inside of the main body and in which the volume of the hollow holes occupying in the main body thereof is greater than or equal to 50%, in a case where the material is prepared as a material at 1 mm square. However, there is no particular limitation. In these materials, the above-described hollow holes on the inside thereof may communicate with each other, or some or every hollow holes may be opened on the surface of the materials.

The average size of hollow holes which a porous body is obtained through observation of a cross-sectional structure of the porous body. First, a cross-sectional structure of a porous body is prepared as a thin slice sample (for example, HE dye sample). Thereafter, a distinct projection portion within the wall formed by macromolecules is connected to the closest projection portion to make the hollow holes distinct. The area of an individual hollow hole which has been obtained and divided is measured, and then, the circle diameter in a case where the area is converted into a circle is calculated. The obtained circle diameter can be set as a size of a hollow hole and the average value of greater than or equal to 20 hollow holes can be set as an average size of hollow holes.

The biocompatible macromolecular porous body of the present invention can be used as a biocompatible macromolecular block (which has been grinded) as will be described below. However, it is possible to evenly hold cells even in a case of using the biocompatible macromolecular porous body as a porous body without grinding. Accordingly, the biocompatible macromolecular porous body of the present invention is also useful as a culture carrier or a cell transplantation carrier.

### (3) Biocompatible Macromolecules

Whether or not the biocompatible macromolecules used in the present invention are decomposed within a living body is not particularly limited as long as the biocompatible macromolecules have affinity to the living body, but the biocompatible macromolecules are preferably formed of a biodegradable material. Specific examples of the biodegradable material include at least one material selected from the group consisting of gelatin, polylactic acid, polyglycolic acid, lactic acid-glycolic acid copolymers (PLGA), hyaluronic acid, glycosaminoglycans, proteoglycans, chondroitin, cellulose, agarose, carboxymethyl cellulose, chitin, and chitosan. Devising of an improvement of cell adhesion properties in these macromolecular materials may be performed. As specific methods thereof, methods such as 1. "coating of the surface of a base material with a cell adhesion substrate (fibronectin, vitronectin, or laminin) or peptides of a cell adhesion sequence (an RGD sequence, an LDV sequence, an REDV sequence, an YIGSR sequence, a PDSGR sequence, an RYVVLPR sequence, an LGTIPG sequence, an RNIAEIIKDI sequence, an IKVAV sequence, an LRE sequence, a DGEA sequence, and a HAV sequence, which are represented by one-letter notation of amino acids)", 2. "aminization or cationization of the surface of a base material", and 3. "plasma treatment performed on the surface of a base material or hydrophilic treatment due to corona discharge" can be used.

Gelatin, collagen, elastin, fibronectin, ProNectin, laminin, tenascin, fibrin, fibroin, entactin, thrombospondin, and RetroNectin are preferable and gelatin, collagen, and atelocollagen are most preferable. As the gelatin to be used in the present invention, natural gelatin or recombinant gelatin is preferable. Recombinant gelatin is more preferable. The natural gelatin referred to herein means gelatin produced using naturally derived collagen. The recombinant gelatin will be described below in the present specification.

A "1/IOB" value which is a hydrophilic value of biocompatible macromolecules used in the present invention is preferably within a range of 0 to 1.0, more preferably within a range of 0 to 0.6, and still more preferably within a range of 0 to 0.4. IOB is an index of hydrophilic and hydrophobic properties based on an organic conceptual diagram representing polarity and non-polarity of an organic compound proposed by Atsushi HUJITA, and the details thereof are described in, for example, "Pharmaceutical Bulletin", vol. 2, 2, pp. 163-173 (1954), "Area of Chemistry" vol. 11, 10, pp. 719-725 (1957), and "Fragrance Journal, vol. 50, pp. 79-82 (1981). Briefly, the root of every organic compound is set to methane (CH₄), and all of other compounds are regarded as derivatives of methane. Certain numerical values for the number of carbons thereof, a substituent group, a transformation portion, a ring, and the like are set, and an organic value (OV) and an inorganic value (IV) are obtained by adding the score thereof. These values are plotted on a diagram in which the organic value is shown on the X-axis and the inorganic value is shown on the Y-axis. IOB in the organic conceptual diagram refers to a ratio of the inorganic value (IV) to the organic value (OV) in the organic conceptual diagram, that is, "inorganic value (IV)/organic value (OV)". The details of the organic conceptual diagram can be referred to "New Edition Organic Conceptual Diagram -Foundation and Application-" (written by Yoshio KOUDA, Sankyo Shuppan Co., Ltd., 2008). In the present specification, the hydrophilic and hydrophobic properties are represented by a "1/IOB" value which was obtained by taking a reciprocal number of IOB. This is a notation of representing more hydrophilic properties as the "1/IOB" value becomes small (close to 0).

The hydrophilic properties and water absorbency become high by making the "1/IOB" value of the macromolecules used in the present invention be within the above-described range, which effectively acts to hold nutrient components. As a result, it is estimated that this point contributes to stability of cells and easy survival of cells in a three-dimensional cell structure (mosaic cell aggregation) of the present invention.

In a case where the biocompatible macromolecules used in the present invention are polypeptides, the hydrophilic and hydrophobic indexes represented by a grand average of hydropathicity (GRAVY) value is preferably -9.0 to 0.3, and more preferably -7.0 to 0.0. The grand average of hydropathicity (GRAVY) value can be obtained through "Gasteiger E., Hoogland C., Gattiker A., Duvaud S., Wilkins M.R., Appel R.D., Bairoch A.; Protein Identification and Analysis Tools on the ExPASy Server; (In) John M. Walker (ed): The Proteomics Protocols Handbook, Humana Press (2005). pp. 571-607" and "Gasteiger E., Gattiker A., Hoogland C., Ivanyi I., Appeal R.D., Bairoch A.; ExPASy: the proteomics server for in-depth protein knowledge and analysis.; Nucleic Acids Res. 31:3784-3788 (2003)".

The hydrophilic properties and water absorbency become high by making the GRAVY value of the macromolecules used in the present invention be within the above-described range, which effectively acts to hold nutrient components. As a result, it is estimated that this point contributes to stability of cells and easy survival of cells in a three-dimensional cell structure (mosaic cell aggregation; cell aggregation in mosaic shape) of the present invention.

The biocompatible macromolecules used in the present invention may be or may not be cross-linked, but are preferably cross-linked. As a general cross-linking method, thermal cross-linking, cross-linking using aldehydes (for example, formaldehyde or glutaraldehyde), cross-linking using a condensation agent (carbodiimide, cyanamide, or the like), enzymatic cross-linking, photocrosslinking, ultraviolet cross-linking, a hydrophobic interaction, hydrogen bonding, an ionic interaction, and the like are known. In the present invention, it is preferable to use a cross-linking method in which glutaraldehyde is not used. As the cross-linking method used in the present invention, thermal cross-linking, ultraviolet cross-linking, or enzymatic cross-linking is more preferable, and thermal cross-link is particularly preferable.

In a case of performing cross-linking using an enzyme, there is no particular limitation as long as the enzyme has a cross-linking action between macromolecular materials. However, it is possible to perform cross-linking preferably using transglutaminase and laccase and most preferably using transglutaminase. Specific examples of protein to be subjected to enzymatic cross-linking using transglutaminase are not particularly limited as long as the protein has a lysine residue and a glutamine residue. Transglutaminase may be derived from a mammal or may be derived from a microorganism. Specific examples thereof include mammal-derived transglutaminase which has been sold as Activa series manufactured by Ajinomoto Co., Inc., and a reagent; guinea pig liver-derived transglutaminase manufactured by, for example, Oriental Yeast Co., Ltd., Upstate USA Inc., or Biodesign International, Inc.; goat-derived transglutaminase; rabbit-derived transglutaminase; and human-derived blood coagulation factors (Factor XIIIa: Haematologic Technologies, Inc).

The reaction temperature when performing cross-linking (for example, thermal cross-linking) without using a cross-linking agent is not particularly limited as long as cross-linking can be performed, but is preferably -100°C to 500°C, more preferably 0°C to 300°C, still more preferably 50°C to 300°C, still more preferably 100°C to 250°C, and still more preferably 120°C to 200°C.

Particularly preferably, it is possible to use recombinant gelatin as biocompatible macromolecules in the present invention. The recombinant gelatin means polypeptides or protein-like substances which have an amino acid sequence similar to that of gelatin produced through gene recombination technology. The recombinant gelatin which can be used in the present invention preferably has a repetition of a sequence (X and Y each independently show any amino acids) represented by Gly-X-Y which is characteristic to collagen (a plurality of pieces of Gly-X-Y may be the same as or different from each other). Preferably, two or more sequences of cell adhesion signals are included in one molecule. As the recombinant gelatin used in the present invention, it is possible to use recombinant gelatin having an amino acid sequence derived from a partial amino acid sequence of collagen, and to use recombinant gelatin disclosed in, for example, EP1014176, US6992172, WO2004/85473A, and WO2008/103041A. However, the recombinant gelatin is not limited thereto. Preferred recombinant gelatin used in the present invention is recombinant gelatin of the following mode.

The recombinant gelatin used in the present invention is excellent in biocompatibility with original performance of natural gelatin, and is excellent in non-infection properties since there is no concern of bovine spongiform encephalopathy (BSE) and the recombinant gelatin with not being naturally derived. In addition, the recombinant gelatin used in the present invention is even compared to natural recombinant gelatin, and a sequence is determined. Therefore, it is possible to accurately design the strength and degradability so as to reduce deviation through cross-linking or the like to be described below.

The molecular weight of recombinant gelatin is preferably 2kDa to 100 kDa, more preferably 2.5 kDa to 95 kDa, more preferably 5 kDa to 90 kDa, and most preferably 10 kDa to 90 kDa.

The recombinant gelatin has a repetition of a sequence represented by Gly-X-Y which is characteristic to collagen. Here, a plurality of pieces of Gly-X-Y may be the same as or different from each other. In Gly-X-Y, Gly represents glycine and X and Y represent an arbitrary amino acid (preferably represents an arbitrary amino acid other than glycine). The GXY sequence characteristic to collagen is a partial structure which is extremely specific compared to other protein in a composition or a sequence of an amino acid of gelatin/collagen. In this section, glycine occupies about one third of the entirety of the amino acid sequence, one sequence is repeated every three sequences. Glycine is the simplest amino acid. Therefore, there is a little restraint in arrangement of molecular chains and glycine significantly contributes to regeneration of a helix structure during gelation. It is preferable that amino acids represented by X and Y contain many imino acids (proline and oxyproline) and occupy 10% to 45% of the entirety of the sequence. It is preferable that preferably 80% or more of the sequence of the amino acids, more preferably 95% or more of the sequence of the amino acids, and most preferably 99% or more of the sequence of the amino acids has a repeating structure of GXY.

In general gelatin, a polar amino acid with an electrical charge and a polar non-charged amino acid exist by 1:1 in polar amino acids. Here, the polar amino acid specifically indicates cysteine, aspartic acid, glutamic acid, histidine, lysine, asparagine, glutamine, serine, threonine, tyrosine, or arginine. Among these, the polar non-charged amino acid indicates cycteine, asparagine, glutamine, serine, threonine, or tyrosine. In recombinant peptides used in the present invention, the proportion of the polar amino acid in the whole constituent amino acid is 10% to 40% and preferably 20% to 30%. It is preferable that the proportion of a non-charged amino acid in the polar amino acid is greater than or equal to 5% and less than 20% and preferably less than 10%. Furthermore, it is preferable that any of serine, threonine, asparagine, tyrosine, and cysteine does not contain one amino acid and preferably two or more amino acids on a sequence.

In general, in polypeptides, minimum amino acid sequences which work as cell adhesion signals are known (for example, Nagai Shoten Co., Ltd., "Pathophysiology", Vol. 9, No. 7 (1990) p. 527). The recombinant gelatin used in the present invention preferably has two or more these cell adhesion signals in one molecule. As the specific sequences, sequences such as an RGD sequence, an LDV sequence, an REDV sequence, an YIGSR sequence, a PDSGR sequence, an RYVVLPR sequence, an LGTIPG sequence, an RNIAEIIKDI sequence, an IKVAV sequence, an LRE sequence, a DGEA sequence, and a HAV sequence, which are represented by one-letter notation of amino acids are preferable in that there are many kinds of cells adhered, an RGD sequence, an YIGSR sequence, a PDSGR sequence, an LGTIPG sequence, an IKVAV sequence, and a HAV sequence are more preferable, and an RGD sequence is particularly preferable. In the RGD sequence, an ERGD sequence is preferable. It is possible to improve the production amount of substrate of a cell using recombinant gelatin having cell adhesion signals. For example, it is possible to improve the production of glycosaminoglycan (GAG) in a case of cartilage differentiation using mesenchymal stem cells as cells.

As arrangement of RGD sequences in recombinant gelatin used in the present invention, it is preferable that the number of amino acids between RGDs is between 0 to 100 and preferably between 25 to 60 without being even.

The content of this minimum amino acid sequence is preferably 3 to 50, more preferably 4 to 30, and particularly preferably 5 to 20 in one molecule of protein in view of cell adhesion properties and proliferation properties. The most preferable content thereof is 12.

In recombinant gelatin used in the present invention, the proportion of RGD motifs with respect to the total number of amino acids is preferably at least 0.4%. In a case where recombinant gelatin contains 350 or more amino acids, each stretch of the 350 amino acids preferably contains at least one RGD motif. The proportion of RGD motifs with respect to the total number of amino acids is still more preferably at least 0.6%, still more preferably at least 0.8%, still more preferably at least 1.0%, still more preferably at least 1.2%, and most preferably at least 1.5%. The number of RGD motifs within a recombinant peptides is, per 250 amino acids, preferably at least 4, still more preferably 6, still more preferably 8, and still more preferably 12 to 16. The proportion of RGD motifs being 0.4% corresponds to at least one RGD sequence per 250 amino acids. The number of RGD motifs is an integer, and therefore, gelatin formed of 251 amino acids needs to contain at least two RGD sequences in order to satisfy the characteristics of 0.4%. It is preferable that the recombinant gelatin of the present invention contains at least two RGD sequences per 250 amino acids, more preferably contains at least three RGD sequences per 250 amino acids, and still more preferably contains at least four RGD sequences per 250 amino acids. As a further mode of the recombinant gelatin of the present invention, the recombinant gelatin contains at least four RGD motifs, preferably 6 RGD motifs, more preferably 8 RGD motifs, and still more preferably 12 to 16 RGD motifs.

In addition, the recombinant gelatin may be partially hydrolyzed.

The recombinant gelatin used in the present invention is preferably represented by Formula: A-[(Gly-X-Y)ₙ]ₘ-B. n pieces of X each independently represent any amino acid and n pieces of Y each independently represent any amino acid. m is preferably 2 to 10 and preferably 3 to 5. n is preferably 3 to 100, more preferably 15 to 70, and most preferably 50 to 65. A represents an arbitrary amino acid or an amino acid sequence, B represents an arbitrary amino acid or an amino acid sequence, n pieces of X each independently represent any amino acid, and n pieces of Y each independently represent any amino acid.

More preferably, the recombinant gelatin used in the present invention is represented by Formula: Gly-Ala-Pro-[(Gly-X-Y)₆₃]₃-Gly (where 63 pieces of X each independently represent any amino acid and 63 pieces of Y each independently represent any amino acid. 63 pieces of Gly-X-Y may be the same as or different from each other).

It is preferable that a plurality of sequence units of collagen which naturally exists are bonded to a repeating unit. Any naturally existing collagen referred to herein may be used as long as the collagen naturally exists, but is preferably a I type, a II type, a III type, a IV type, and a V type, and more preferably a I type, a II type, and a III type. According to another form, the collagen is preferably derived from a human, cattle, a pig, a mouse, or a rat. The collagen is more preferably derived from a human.

An isoelectric point of the recombinant gelatin used in the present invention is preferably 5 to 10, more preferably 6 to 10, and still more preferably 7 to 9.5.

It is preferable that the recombinant gelatin is not deaminated.

It is preferable that the recombinant gelatin does not have telopeptide.

It is preferable that the recombinant gelatin is substantially a pure collagen material which is prepared using a nucleic acid encoding natural collagen.

It is preferable that the recombinant gelatin used in the present invention is any of
(a) protein having an amino acid sequence described in SEQ ID No: 1;
(b) protein which has an amino acid sequence in which one or a plurality of amino acids are deleted, substituted, or added in the amino acid sequence described in SEQ ID No: 1, and has biocompatibility; or
(c) protein which has an amino acid sequence having 80% or more (more preferably 90% or more and still more preferably 95% or more) homology to the amino acid sequence described in SEQ ID No: 1, and has biocompatibility.

It is more preferable that the recombinant gelatin used in the present invention is any of
(a) protein formed of an amino acid sequence described in SEQ ID No: 1;
(b) protein which is formed of an amino acid sequence in which one or a plurality of amino acids are deleted, substituted, or added in the amino acid sequence described in SEQ ID No: 1, and has biocompatibility; or
(c) protein which has an amino acid sequence having 80% or more (more preferably 90% or more and still more preferably 95% or more) homology to the amino acid sequence described in SEQ ID No: 1, and has biocompatibility.
"one or a plurality of" in the "amino acid sequence in which one or a plurality of amino acids are deleted, substituted, or added" preferably means 1 to 20 amino acids, more preferably means 1 to 10 amino acids, still more preferably means 1 to 5 amino acids, and particularly preferably means 1 to 3 amino acids.

The recombinant gelatin used in the present invention can be produced through gene recombination technology which is known to those skilled in the art, and can be produced in accordance with, for example, methods disclosed in EP1014176A2, US6992172, WO2004-85473A, WO2008/103041A, and the like. Specifically, a gene encoding an amino acid sequence of predetermined recombinant gelatin is acquired, the acquired gene is incorporated into an expression vector to produce a recombinant expression vector, and a transformant is produced by introducing the recombinant expression vector into an appropriate host. The recombinant gelatin is produced by culturing the obtained transformant in an appropriate medium. Therefore, it is possible to prepare the recombinant gelatin used in the present invention by collecting the recombinant gelatin produced from a culture product.

### (4) Biocompatible Macromolecular Block

The method for producing a biocompatible macromolecular block (aggregation containing biocompatible macromolecules) is not particularly limited. For example, it is possible to obtain a biocompatible macromolecular block by grinding a porous body of biocompatible macromolecules using a grinder (such as new PowerMILL).

The shape of the biocompatible macromolecular block in the present invention is not particularly limited.

The size of one biocompatible macromolecular block in the present invention is preferably 20 µm to 200 µm, more preferably 20 µm to 120 µm, still more preferably 50 µm to 120 µm. It is possible to achieve more excellent cell viability by making the size of one biocompatible macromolecular block is within the above-described range. The size of one biocompatible macromolecular block does not mean that an average value of the sizes of a plurality of biocompatible macromolecular blocks is within the above-described range, but means the size of each biocompatible macromolecular block which is obtained by sieving a plurality of biocompatible macromolecular blocks.

As will be described in the present specification, it is possible to produce a cell structure of the present invention by mixing the biocompatible macromolecular block of the present invention with at least one kind of cell. That is, the biocompatible macromolecular block of the present invention is useful as a reagent for producing a cell structure of the present invention.

### (5) Cells

As cells used in the present invention, it is possible to use arbitrary cells as long as cell transplantation for the purpose of a cell structure of the present invention can be performed, and the kinds thereof are not particularly limited. In addition; one kind of cell may be used or a plurality of kinds of cells may be used in combination. In addition, cells to be used are preferably animal cells, more preferably vertebrate animal-derived cells, and particularly preferably human-derived cells. The kinds of vertebrate animal-derived cells (particularly human-derived cells) may be either of pluripotent cells, somatic stem cells, precursor cells, and mature cells. As the pluripotent cells, it is possible to use, for example, ES cells, GS cells, or iPS cells. As the somatic stem cells, it is possible to use, for example, mesenchymal stem cells (MSC), hematopoietic stem cells, amniotic cells, cord blood cells, bone marrow-derived cells, cardiac muscle stem cells, adipose-derived stem cells, or neural stem cells. As the precursor cells and mature cells, it is possible to use, for example, cells derived from the skin, the dermis, the epidermis, muscles, cardiac muscles, nerves, bones, cartilage, the endothelium, the brain, epithelium, the heart, the kidney, the liver, the pancreas, the spleen, the inside of the oral cavity, the cornea, bone marrow, cord blood, amnion, or hair. As the human-derived cells, it is possible to use, for example, ES cells, iPS cells, MSC, chondrocytes, osteoblasts, osteoprogenitor cells, mesenchyme cells, myoblasts, cardiac muscle cells, cardiac myoblasts, nerve cells, liver cells, beta cells, fibroblasts, corneal endothelial cells, vascular endothelial cells, corneal epithelial cells, amniotic cells, cord blood cells, bone marrow-derived cells, or hematopoietic stem cells. In addition, the cells may be derived from any of autologous cells or heterologous cells.

For example, in heart disease such as serious heart failure or serious myocardial infarction it is possible to favorably use myocardial cells, smooth muscle cells, fibroblasts, skeletal muscle-derived cells (particularly satellite cells), bone marrow cells (particularly bone marrow cells that have been differentiated into cardiac muscle-like cells), all of which are removed in autologous and heterologous manners. Furthermore, it is possible to appropriately select transplantation cells even in other organs. Examples of the transplantation include transplantation of nerve precursor cells or cells which can be differentiated into nerve cells, into a cerebral ischemia site or a stroke site or transplantation of vascular endothelial cells, or cells which can be differentiated into the vascular endothelial cells, into a myocardial infarction site or a skeletal muscle ischemia site.

In addition, examples of the cells include cells used in cell transplantation with respect to diabetic organopathy. Examples thereof include various cells for cell transplantation therapy which have been reviewed, with respect to diseases such as kidney disorders, pancreas disorders, peripheral nerve disorders, eye disorders, and interruption in circulation of the limbs. That is, an attempt of transplanting insulin-secreting cells into the pancreas of which the insulin-secreting ability is deteriorated, transplantation of bone marrow-derived cells with respect to interruption in circulation of the limbs, or the like has been reviewed, and it is possible to use such cells.

In addition, it is possible to use vascular cells. In the present specification, the vascular cells mean cells associated with angiogenesis, and are cells constituting blood vessels and blood, and precursor cells and somatic stem cells which can be differentiated to the cells thereof. Here, pluripotent cells such as ES cells, GS cells, or iPS cells; or cells, such as mesenchymal stem cells (MSC), constituting blood vessels and blood, which are not naturally differentiated are not contained in the vascular cells. As the vascular cells, cells constituting blood vessels are preferable. In the vertebrate animal-derived cells (particularly human-derived cells), favorable examples of the cells constituting blood vessels include vascular endothelial cells and vascular smooth muscle cells. As the vascular endothelial cells, both of venous endothelial cells and arterial endothelial cells are included. As the precursor cells of the vascular endothelial cells, it is possible to use vascular endothelial precursor cells, and to preferably use vascular endothelial cells and vascular endothelial precursor cells. As the cells constituting blood, it is possible to use corpuscle cells. It is possible to use white corpuscle cells such as lymphocytes or neutrophils, monocyte cells, and hematopoietic stem cells which are stem cells thereof.

In the present specification, the non-vascular cells mean cells other than the above-described vascular cells. For example, ES cells, iPS cells, mesenchymal stem cells (MSC), cardiac muscle stem cells, cardiac muscle cells, fibroblasts, myoblasts, chondrocytes, myoblasts, liver cells, or nerve cells can be used. Mesenchymal stem cells (MSC), chondrocytes, myoblasts, cardiac muscle stem cells, cardiac muscle cells, liver cells, or iPS cells can be preferably used. Mesenchymal stem cells (MSC), cardiac muscle stem cells, cardiac muscle cells, or myoblasts are more preferably used.

### (6) Cell Structure

The cell structure of the present invention is a cell structure including the biocompatible macromolecular block according to the above-described present invention; and at least one kind of cell, in which a plurality of the biocompatible macromolecular blocks are arranged in a gap between a plurality of cells. The cell structure of the present invention can be used for cell transplantation.

In the present invention, the structure can have a thickness suitable for cell transplantation by three-dimensionally arranging the plurality of macromolecular blocks in the gap between the plurality of cells in a mosaic shape using the above-described biocompatible macromolecular blocks and the above-described cells. Moreover, a three-dimensional cell structure in which cells evenly exist in the structure is formed by three-dimensionally arranging the biocompatible macromolecular blocks and the cells in a mosaic shape, and it is possible to deliver nutrients to the inside of the three-dimensional cell structure from the outside.

In the cell structure of the present invention, the plurality of macromolecular blocks are arranged in a gap between the plurality of cells. Here, the "gap between cells" is not necessarily a space closed by the constituent cells, and may be interposed by the cells. Gaps are not necessarily present between all of the cells, and there may be a place where cells are brought into contact with each other. The distance of a gap between cells through macromolecular blocks, that is, the gap distance when selecting a certain cell, and a cell existing in a shortest distance from the certain cell is not particularly limited. However, the distance is preferably the size of a macromolecular block, and a favorable distance is also within the range of the favorable size of a macromolecular block.

In addition, the macromolecular blocks according to the present invention have a configuration of being interposed by the cells. However, there are not necessarily cells between all of the macromolecular blocks, and there may be a place where macromolecular blocks are brought into contact with each other. The distance between macromolecular blocks through cells, that is, the distance when selecting a macromolecular block, and a macromolecular block existing in a shortest distance from the macromolecular block is not particularly limited. However, the distance is preferably the size of an aggregation of cells when one or several cells to be used are gathered. For example, the size thereof is 10 µm to 1000 µm, preferably 10 µm to 100 µm, and more preferably 10 µm to 50 µm.

The expressions such as "evenly exist", for example, the "three-dimensional cell structure in which cells evenly exist in the structure" is used in the present specification. However, the expression does not mean complete evenness, but means that it is possible to deliver nutrients to the inside of the three-dimensional cell structure from the outside, which is an action and an effect of the present invention.

The thickness or the diameter of the cell structure of the present invention can be set to a desired thickness. As the lower limit, greater than or equal to 215 µm is preferable, greater than or equal to 400 µm is more preferable, and greater than or equal to 730 µm is most preferable. The upper limit of the thickness or the diameter is not particularly limited, but as a general range in use is preferably less than or equal to 3 cm, more preferably less than or equal to 2 cm, and still more preferably less than or equal to 1 cm. In addition, the range of the thickness or the diameter of the cell structure is preferably 400 µm to 3 cm, more preferably 500 µm to 2 cm, and still more preferably 720 µm to 1 cm.

In the cell structure of the present invention, a region formed of macromolecular blocks and a region formed of cells are preferably arranged in a mosaic shape. The "thickness or the diameter of cell structure" in the present specification indicates the following. When selecting a certain point A in the cell structure, the length of a line segment which divides the cell structure is set as a line segment A such that the distance from the external boundary of the cell structure becomes shortest within a straight line passing through the point A. A point A at which the line segment A thereof in the cell structure becomes longest is selected, and the length of the line segment A during the selection thereof is set as the "length or the diameter of the cell structure".

In addition, in a case of using the cell structure of the present invention as a cell structure before merging performed through the method for producing a cell structure of the present invention to be described below, or as a cell structure before adding a second macromolecular block, the range of the thickness or the diameter of the cell structure is preferably 10 µm to 1 cm, more preferably 10 µm to 2000 µm, still more preferably 15 µm to 1500 µm, and most preferably 20 µm to 1300 µm.

In the cell structure of the present invention, the ratio of a macromolecular block to a cell is not particularly limited. However, it is preferable that the ratio of a macromolecular block per cell is preferably 0.0000001 µg to 1 µg, still more preferably 0.000001 µg to 0.1 µg, more preferably 0.00001 µg to 0.01 µg, and most preferably 0.00002 µg to 0.006 µg. It is possible to make cells more evenly exist by making the ratio thereof be within the above-described range. In addition, it is possible to exhibit an effect of the cells when using the cells for the above-described purpose, by making the lower limit be within the above-described range. Moreover, it is possible to supply components in arbitrarily existing macromolecular blocks to cells, by making the upper limit be within the above-described range. Here, the components in macromolecular blocks are not particularly limited, but examples thereof include components contained in a medium to be described below.

In addition, the cell structure of the present invention may contain an angiogenic factor. Here, examples of the angiogenic factor favorably include a basic fibroblast growth factor (bFGF), a vascular endothelial growth factor (VEGF), and a hepatocyte growth factor (HGF). The method for producing a cell structure containing a angiogenic factor is not particularly limited, but examples thereof include a production method using macromolecular blocks in which a angiogenic factor is impregnated. It is preferable that the cell structure of the present invention contains a angiogenic factor from the viewpoint of promoting angiogenesis.

As the cell structure of the present invention, it is possible to favorably use a cell structure containing non-vascular cells. In addition, it is possible to favorably use a cell structure in which cells constituting the cell structure of the present invention are only non-vascular cells. It is possible to form a blood vessel in a transplantation site after the transplantation using the cell structure of the present invention which contains only non-vascular cells as cells. In addition, in a case where the cells constituting the cell structure of the present invention are two or more kinds and contain both of the non-vascular cells and vascular cells, it is possible to form more blood vessels compared to the case where the cell structure is constituted only non-vascular cells, which is preferable.

Furthermore, in a case where the cells constituting the cell structure of the present invention are two or more kinds and have a region in which the area of the vascular cells in the central portion of the cell structure is larger than that of the vascular cells in the peripheral portion, it is possible to form more blood vessels, which is more preferable. Here, the "possession of a region in which the area of the vascular cells in the central portion of the cell structure is larger than that of the vascular cells in the peripheral portion" specifically refers that there is a sample having the above-described region when an arbitrary thin slice sample with 2 µm is produced. Here, the central portion of the cell structure refers to the area of the distance of 80% from the center, in the distance from the center to the surface of the cell structure. Moreover, the peripheral portion of the cell structure refers to the area from a place of 80% from the center to the surface of the structure. The central portion of the cell structure is determined as follows.

In an arbitrary cross section passing through the center of a cell structure, a radius X, in which the area of a portion in which the center of a circle of the radius X is moved by one rotation along the outer extension of the cross section, and from which a portion where the moved circle and the cross section overlap each other is removed becomes 64% of the cross-sectional area of the cross section, is obtained. The center of the circle of the radius X is moved by one rotation, and the portion in which the center of a circle of the radius X is moved by one rotation and from which a portion where the moved circle and the cross section overlap each other is removed, is set as a central portion of the cell structure. At this time, a cross section in which the cross-sectional area becomes largest is most preferable. In the cross section in which the cross-sectional area becomes largest, a radius Y, in which a portion in which the center of a circle of the radius Y is moved by one rotation along the outer extension of the cross section, and from which a portion where the moved circle and the cross section overlap each other is removed is determined at one point, is obtained. The one point in which the center of a circle of the radius Y is moved by one rotation and from which a portion where the moved circle and the cross section overlap each other is removed, is set as the center of the cell structure. In a case where the portion becomes a line segment without being determined at one point or in a case where there are a plurality of line segments, a point at which the length of each line segment is divided equally in half is set as the center.

Specifically, the cell structure has a region in which the proportion of the vascular cells in the central portion is preferably 60% to 100%, more preferably 65% to 100%, still more preferably 80% to 100%, and still more preferably 90% to 100%, with respect to the total area of the vascular cells. Here, the possession of a region in which the proportion of the vascular cells in the central portion is 60% to 100% with respect to the total area of the vascular cells specifically refers that there is a sample having an area with the proportion when an arbitrary thin slice sample with 2 µm is produced. The angiogenesis can be more promoted by making the proportion be within the range.

The proportion of the vascular cells in the central portion can be calculated by, for example, obtaining an average value of color densities (strengths) in the central portion using image treatment software ImageJ after dyeing the vascular cells to be measured, when producing a thin slice sample; calculating the area x the strength of the central portion; further obtaining an average value of all color densities (strengths); calculating all of the area x the strength; and obtaining the proportion of the area x the strength of the central portion with respect to all of the area x the strength. Here, as the method for dyeing the vascular cells, any well-known dyeing method can be appropriately used. For example, it is possible to use a CD31 antibody in a case of using human vascular endothelial precursor cells (hECFC) as cells.

In addition, it is preferable that the cell structure has a region in which the density of the vascular cells in the central portion of the cell structure is greater than or equal to 1.0 x 10⁻⁴ cells/µm³. It is more preferable that the cell density of the cell structure becomes the cell density in the entirety of the central portion of the cell structure. Here, the possession of a region of greater than or equal to 1.0 x 10⁻⁴ cells/µm³ specifically refers that there is a sample having the region with the density when an arbitrary thin slice sample with 2 µm is produced. The cell density is more preferably 1.0 x 10⁻⁴ cells/µm³ to 1.0 x 10⁻³ cells/µm³, still more preferably 1.0 x 10⁻⁴ cells/µm³ to 2.0 x 10⁻⁴ cells/µm³, still more preferably 1.1 x 10⁻⁴ cells/µm³ to 1.8 x 10⁻⁴ cells/µm³, and still more preferably 1.4 x 10⁻⁴ cells/µm³ to 1.8 x 10⁻⁴ cells/µm³. The angiogenesis can be more promoted by making the cell density be within the range.

Here, the density of the vascular cells in the central portion can be obtained by actually counting the number of cells of a thin slice sample and dividing the number of cells by the volume. Here, the central portion is determined as follows. The portion of the thickness of the thin slice sample in the vertical direction of the above-described central portion is regarded as a cut-off portion. As the method for obtaining the cell density, for example, it is possible to calculate the number of vascular cells in the central portion by overlapping a thin slice sample, in which the above-described vascular cells to be measured are dyed, and a thin slice sample, in which a cell nuclei is dyed, each other, and counting the number of the overlapping cell nuclei. The volume can be obtained by obtaining the area of the central portion using ImageJ and multiplying the thickness of the thin slice sample thereof.

The cell structure of the present invention contains two or more kinds of cells, and a cell structure, in which blood vessels are formed using the cell structure of the present invention containing both of the non-vascular cells and vascular cells, is also included. Here, the favorable range of the "two or more kinds of cells and the cell structure of the present invention containing both of the non-vascular cells and vascular cells" is the same as that described above. Examples of the method for constructing blood vessels include a method for pasting a cell sheet, which is mixed with vascular cells, to a gel material in which the vascular portion is hollowed out in a tunnel shape, and culturing the gel material while allowing a culture solution to flow to the tunnel. In addition, it is also possible to construct the blood vessels by putting vascular cells between a cell sheet in a sandwich shape.

### (7) Method for Producing Cell Structure

The cell structure of the present invention can be produced by mixing a biocompatible macromolecular block of the present invention with at least one kind of cell. More specifically, the cell structure of the present invention can be produced by alternately arranging a biocompatible macromolecular block (an aggregation formed of biocompatible macromolecules) and a cell. The production method is not particularly limited, but a method for sowing cells after forming a macromolecular block is preferably used. Specifically, it is possible to produce the cell structure of the present invention by incubating a mixture of a biocompatible macromolecular block and a cell-containing culture solution. For example, in the solution held by a container, in the container, a cell and the previously produced biocompatible macromolecular block are arranged in a mosaic shape. It is preferable to promote and control the formation of the arrangement, which is formed of a cell and a biocompatible base material, in a mosaic shape, through natural aggregation, natural fall, centrifugation, or agitation as means for the arrangement.

As the container to be used, a container formed of a low-adhesive cell material or a non-adhesive cell material is preferable and a container formed of polystyrene, polypropylene, polyethylene, glass, polycarbonate, or polyethylene terephthalate is more preferable. The shape of the bottom surface of a container is preferably a flat bottom shape, a U shape, and a V shape.

The mosaic-like cell structure obtained through the above-described method can be produced so as to have a desired size through, for example,
(a) merging mosaic-like cell aggregations, which have been separately adjusted, with each other, or
(b) increasing the volume of the structure under a differentiation medium or a proliferation medium.

The method for merging the aggregations or the method for increasing the volume of the structure is not particularly limited.

For example, it is possible to increase the volume of the cell structure by exchanging a medium with a differentiation medium or a proliferation medium in a process of incubating a mixture of a biocompatible macromolecular block and a cell-containing culture solution. Preferably, it is possible to produce a cell structure in which cells evenly exist in the cell structure and which has a desired size, by further adding a biocompatible macromolecular block, in the process of incubating a mixture of a biocompatible macromolecular block and a cell-containing culture solution.

The method for merging mosaic-like cell aggregations, which have been separately adjusted, with each other is specifically a method for producing cell structures, the method including a process of merging a plurality of cell structures which include a plurality of biocompatible macromolecular blocks and a plurality of cells and in which one or a plurality of the biocompatible macromolecular blocks are arranged in some or all of a plurality of gaps formed by the plurality of cells.

Favorable ranges of the "(kinds, sizes, or the like) of biocompatible macromolecular blocks", the "cells", the "gap between the cells", the "(size or the like) of the obtained cell structure", the "ratio of the cells to the macromolecular blocks", and the like according to the method for producing a cell structure of the present invention are the same as those described in the present specification.

In addition, it is preferable that the thickness or the diameter of each cell structure before the merging is 10 µm to 1 cm, and the thickness or the diameter thereof after the merging is 400 µm to 3 cm. Here, the thickness or the diameter of each cell structure before the merging is more preferably 10 µm to 2000 µm, still more preferably 15 µm to 1500 µm, and most preferably 20 µm to 1300 µm. In addition, the range of the thickness or the diameter thereof after the merging is more preferably 500 µm to 2 cm and still more preferably 720 µm to 1 cm.

The above-described method for producing a cell structure having a desired size by further adding a biocompatible macromolecular block is specifically a method for producing cell structures, the method including a process of performing incubating after further adding a second biocompatible macromolecular block to the cell structures which include a plurality of first biocompatible macromolecular blocks and a plurality of cells and in which one or a plurality of the biocompatible macromolecular blocks are arranged in some or all of a plurality of gaps formed by the plurality of cells. Here, favorable ranges of the "(kinds, sizes, or the like) of biocompatible macromolecular blocks", the "cells", the "gap between the cells", the "(size or the like) of the obtained cell structure", the "ratio of the cells to the macromolecular blocks", and the like are the same as those described in the present specification.

Here, cell structures which need to be merged with each other are preferably installed in a distance of 0 µm to 50 µm, more preferably installed in a distance of 0 µm to 20 µm, and still more preferably installed in a distance of 0 µm to 5 µm. It is considered that, when merging cell structures with each other, cells or a substrate produced by the cells play a role of an adhesive agent due to proliferation and extension of cells, and the cell structures are bonded to each other. Therefore, it is easy to bond the cell structures to each other by making the distance thereof be within the above-described range.

The range of the thickness or the diameter of the cell structure obtained through the method for producing a cell structure of the present invention is preferably 400 µm to 3 cm, more preferably 500 µm to 2 cm, and still more preferably 720 µm to 1cm.

It is preferable to appropriately select pace for further adding a second biocompatible macromolecular block when performing incubating after further adding the second biocompatible macromolecular block in accordance with the proliferation rate of cells to be used. Specifically, if the pace for adding a second biocompatible macromolecular block is fast, cells move to the outside of a cell structure, and therefore, the evenness of the cells is deteriorated. If the pace for adding the second biocompatible macromolecular block is slow, a place in which the proportion of cells is increased can be generated, and therefore, evenness of the cells is deteriorated. Thus, the pace is selected in consideration of the proliferation rate of cells to be used.

Favorable examples of the method for producing a cell structure in a case of containing both of the non-vascular cells and vascular cells include the following production methods (a) to (c).
(a) is a production method including a process of adding a vascular cell and a biocompatible macromolecular block after forming a cell structure through the above-described method using a non-vascular cell. Here, the "process of adding a vascular cell and a highly biocompatible molecular block" includes all of the above-described method for merging mosaic-like cell aggregations, which have been adjusted, with each other and the above-described method for increasing the volume of the structure under a differentiation medium or a proliferation medium. According to these methods, it is possible to produce (i) a cell structure of which the area of non-vascular cells in the central portion of the cell structure is larger compared to that of vascular cells, and the area of vascular cells in the peripheral portion is larger compared to that of non-vascular cells; (ii) a cell structure of which the area of non-vascular cells in the central portion of the cell structure is larger than that of non-vascular cells in the peripheral portion; and (iii) a cell structure of which the area of vascular cell in the central portion of the cell structure is smaller than that of vascular cells in the peripheral portion.
(b) is a production method including a process of adding a non-vascular cell and a biocompatible macromolecular block after forming a cell structure through the above-described method using a vascular cell. Here, the "process of adding a non-vascular cell and a biocompatible macromolecular block" includes all of the above-described method for merging mosaic-like cell aggregations, which have been adjusted, with each other and the above-described method for increasing the volume of the structure under a differentiation medium or a proliferation medium. According to these methods, it is possible to produce (i) a cell structure of which the area of vascular cells in the central portion of the cell structure is larger compared to that of non-vascular cells, and the area of non-vascular cells is larger compared to that of vascular cells in the peripheral portion; (ii) a cell structure of which the area of vascular cells in the central portion of the cell structure is larger than that of vascular cells in the peripheral portion; and (iii) a cell structure of which the area of non-vascular cell in the central portion of the cell structure is smaller than that of non-vascular cells in the peripheral portion.
(c) is a production method in which a cell structure is formed through the above-described method substantially using non-vascular cells and vascular cell at the same time. In this method, it is possible to produce a cell structure in which in all regions of the cell structure, neither of the non-vascular cells nor the vascular cells is not greatly unevenly distributed.

From the viewpoint of forming blood vessels in a transplantation site after the transplantation, the cell structure is preferably a cell structure of which the area of vascular cells in the central portion of the cell structure is larger compared to that of non-vascular cells and the area of non-vascular cells in the peripheral portion is larger compared to that of vascular cells; or a cell structure of which the area of vascular cells in the central portion is larger than that of vascular cells in the peripheral portion. It is possible to further promote angiogenesis by setting the cell structures. Furthermore, a cell structure having a large number of cells existing in the central portion in the cell structures can further promote angiogenesis.

For the same reason, the production method including a process of adding a non-vascular cell and a biocompatible macromolecular block after forming the cell structure using the vascular cell is preferable. Moreover, it is more preferable that the number of vascular cell is made to large.

### (8) Application of Cell Structure

The cell structure of the present invention can be used for the purpose of, for example, cell transplantation in a heart disease site caused by serious heart failure, serious myocardial infarction, or the like, or in a disease site caused by cerebral ischemia or stroke. In addition, it is also possible to use the cell structure of the present invention for diseases such as diabetic renal disorders, pancreas disorders, peripheral nerve disorders, eye disorders, and interruption in circulation of the limbs. As the transplantation method, it is possible to use a method using incision, injection, or an endoscope. In the cell structure of the present invention, it is possible to reduce the size of the structure unlike a cell-transplanted substance such as a cell sheet, and therefore, it is possible to perform less invasive transplantation method such as transplantation performed through injection.

In addition, according to the present invention, there is provided a cell transplantation method. The cell transplantation method of the present invention uses the cell structure of the present invention described above. The favorable range of the cell structure is the same as that described above.

The present invention will be more specifically described using the following Examples, but is not limited by Examples.

### Examples

### [Material and Container]

### [Recombinant Peptides (Recombinant Gelatin)]

CBE (which is disclosed in WO2008-103041A) described in the following was prepared as recombinant peptides (recombinant gelatin).
CBE3
Molecular weight: 51.6 kD
Structure: GAP[(GXY)₆₃]₃G
Number of amino acids: 571
RGD sequence: 12
Imino acid content: 33%

Almost 100% of amino acids have a repeating structure of GXY. In the amino acid sequence of CBE3, serine, threonine, asparagine, tyrosine, and cysteine are not included. CBE3 has an ERGD sequence.
Isoelectric point: 9.34, GRAVY value: -0.682, 1/IOB value: 0.323

Amino acid sequence (SEQ ID No: 1 in a sequence table) (which is the same as that of SEQ ID No: 3 in WO2008/103041A. However, X in the end is corrected to "P").

### [PTFE Thickness·Cylindrical Container]

A cylindrical cup-shaped polytetrafluoroethylene (PTFE)container with a low surface thickness of 3 mm, a diameter of 51 mm, a side surface thickness of 8 mm, and a thickness of 25 mm was prepared. When the curved surface of the cylindrical cup is set as a side surface, the side surface of the cylindrical cup is closed by PTFE with 8 mm and the bottom surface (circular shape of a flat plate) is also closed by PTFE with 3 mm. In contrast, the upper surface is in an open shape. Accordingly, the inner diameter of the cylindrical cup is set to 43 mm. Hereinafter, this container is called a PTFE thickness·cylindrical container.

### [Aluminum Glass·Cylindrical Container]

A cylindrical cup-like aluminum container with a thickness of 1 mm and a diameter of 47 mm was prepared. When the curved surface of the cylindrical cup is set as a side surface, the side surface of the cylindrical cup is closed by aluminum with 1 mm and the bottom surface (circular shape of a flat plate) is also closed by aluminum with 1 mm. In contrast, the upper surface is in an open shape. In addition, Teflon (registered trademark) with a thickness of 1 mm was evenly spread over only the inside of the side surface, and as a result, the inner diameter of the cylindrical cup became 45 mm. In addition, the bottom surface of this container is in a state in which a glass plate with 2.2 mm outer tube aluminum was adhered thereto. Hereinafter, this container is called an aluminum glass·cylindrical container.

### [Example 1]

### [Freezing Process with Small Temperature Difference and Drying Process]

An aqueous CBE3 solution was made to flow into a PTFE thickness·cylindrical container and an aluminum glass·cylindrical container and was cooled down from the bottom surface within a vacuum freeze dryer (TF5-85ATNNN: Takara Co., Ltd.) using a cooling shelf.

A combination of settings for the final concentration of the container and the aqueous CBE3 solution, the liquid amount, and the temperature of the shelf at that is time was prepared as described below.

### "A" Final Concentration of PTFE Thickness·Cylindrical Container and Aqueous CBE3 Solution Being 4 Mass% and Amount of Aqueous Solution Being 4 mL

As the setting of the temperature of a shelf, the temperature was cooled down until the temperature becomes -10°C, and then, freezing was performed for 1 hour at -10°C, for 2 hours at -20°C, for 3 hours at -40°C, and finally for 1 hour at -50°C. Thereafter, this frozen product was subjected to vacuum drying for 24 hours at -20°C after the setting of the temperature of the shelf was returned to -20°C. After 24 hours, the temperature of the shelf was increased to 20°C in a state in which the vacuum drying was continued as it was, and the vacuum drying was further performed for 48 hours at 20°C until the vacuum degree was sufficiently decreased (1.9 x 10⁵ Pa). Then, the product was taken out of the vacuum freeze dryer. Accordingly, a porous body was obtained.

### "B" Final Concentration of Aluminum·Glass·Cylindrical Container and Aqueous CBE3 solution Being 4 Mass% and Amount of Aqueous Solution Being 4 mL

As the setting of the temperature of a shelf, the temperature was cooled down until the temperature becomes -10°C, and then, freezing was performed for 1 hour at -10°C, for 2 hours at -20°C, for 3 hours at -40°C, and finally for 1 hour at -50°C. Thereafter, this frozen product was subjected to vacuum drying for 24 hours at -20°C after the setting of the temperature of the shelf was returned to -20°C. After 24 hours, the temperature of the shelf was increased to 20°C in a state in which the vacuum drying was continued as it was, and the vacuum drying was further performed for 48 hours at 20°C until the vacuum degree was sufficiently decreased (1.9 x 10⁵ Pa). Then, the product was taken out of the vacuum freeze dryer. Accordingly, a porous body was obtained.

### "C" Final Concentration of PTFE Thickness·Cylindrical Container and Aqueous CBE3 Solution Being 4 Mass% and Amount of Aqueous Solution Being 10 mL

As the setting of the temperature of a shelf, the temperature was cooled down until the temperature becomes -10°C, and then, freezing was performed for 1 hour at -10°C, for 2 hours at -20°C, for 3 hours at -40°C, and finally for 1 hour at -50°C. Thereafter, this frozen product was subjected to vacuum drying for 24 hours at -20°C after the setting of the temperature of the shelf was returned to -20°C. After 24 hours, the temperature of the shelf was increased to 20°C in a state in which the vacuum drying was continued as it was, and the vacuum drying was further performed for 48 hours at 20°C until the vacuum degree was sufficiently decreased (1.9 x 10⁵ Pa). Then, the product was taken out of the vacuum freeze dryer. Accordingly, a porous body was obtained.

### [Comparative Example 1]

### [Freezing Process and Drying Process with Large Temperature Difference]

4 mL of an aqueous CBE3 solution was made to flow into an aluminum glass·cylindrical container and was cooled down from the bottom surface within a freezer using a cooling shelf. A combination of settings for the final concentration of the container and the aqueous CBE3 solution, the liquid amount, and the temperature of the shelf at that is time was prepared as described below. In addition, freezing was performed by installing the aqueous CBE3 solution-contained container in a place which had been sufficiently cooled down until the temperature of the shelf reached a previously set temperature.

### "D" Final Concentration of Aluminum Glass·Cylindrical Container and Aqueous CBE3 Solution Being 4 Mass% and Amount of Aqueous Solution Being 4 mL

A aqueous CBE3 solution-contained aluminum glass·container was placed in a place of which the temperature of a shelf was set to be previously cooled down to -60°C, and freezing was performed while maintaining the temperature of the shelf at -60°C as it was. Thereafter, freeze-drying was performed on this frozen product to obtain a porous body.

### "E" Final Concentration of Aluminum Glass·Cylindrical Container and Aqueous CBE3 Solution Being 7.5 Mass% and Amount of Aqueous Solution Being 4 mL

A aqueous CBE3 solution-contained aluminum glass·container was placed in a place of which the temperature of a shelf was set to be previously cooled down to -60°C, and freezing was performed while maintaining the temperature of the shelf at -60°C as it was. Thereafter, freeze-drying was performed on this frozen product to obtain a porous body.

### "F" Final Concentration of Aluminum Glass-Cylindrical Container and Aqueous CBE3 Solution Being 4 Mass% and Amount of Aqueous Solution Being 4 mL

A aqueous CBE3 solution-contained aluminum glass·container was placed in a place of which the temperature of a shelf was set to be previously cooled down to -40°C, and freezing was performed while maintaining the temperature of the shelf at -40°C as it was. Thereafter, freeze-drying was performed on this frozen product to obtain a porous body.

### [Example 2]

### [Measurement of Temperature Difference in Each Freezing Process]

With respect to each of "A" to "F", the liquid temperature of the surface of water in the central portion of a circle within a container was measured as a liquid temperature (a liquid temperature of the non-cooled surface) of a place which is farthest from a cooling side within a solution and the liquid temperature of the bottom portion within the container was measured as a liquid temperature (liquid temperature of the cooled surface) of a place which is closest to the cooling side within the solution.

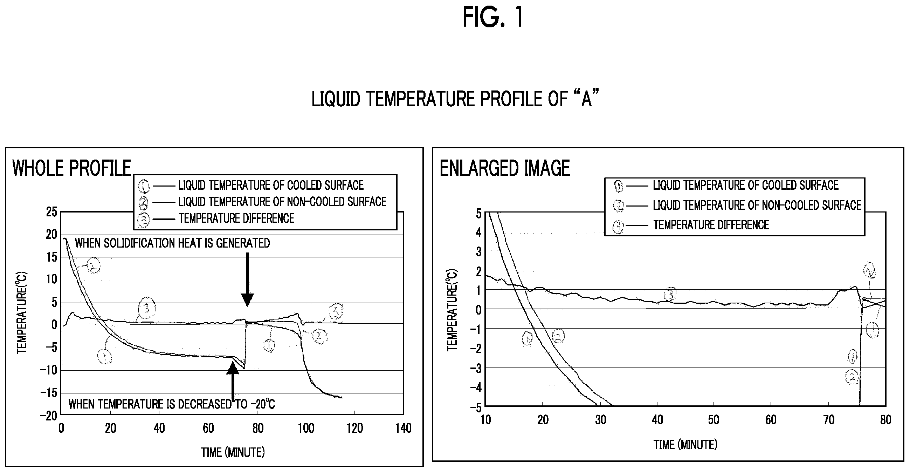

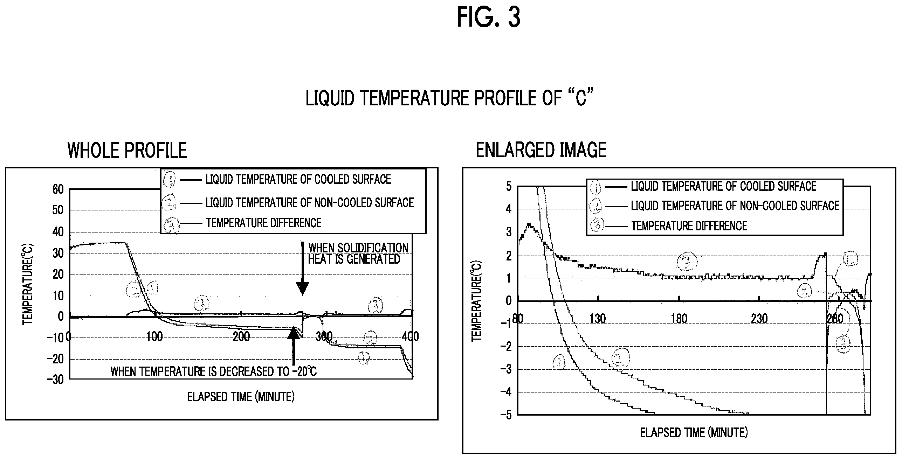

As a result, each temperature and each profile of the temperature difference thereof were as in each of Figs. 1 to 6.

In "A", "B", and "C" from Figs. 1 to 3, it was found that the liquid temperature in a section, in which the temperature of a shelf was set to -10°C (before decreasing the temperature to -20°C), was below 0°C as a melting point, and in the state, a product was in an (unfrozen·supercooled) state in which freezing did not occur. In addition, in this state, the temperature difference between the liquid temperature of the cooled surface and the liquid temperature of the non-cooled surface became lower than or equal to 2.5°C. Thereafter, a timing, at which the liquid temperature is rapidly increased to around 0°C by further decreasing the temperature of the shelf to -20°C, was confirmed. Here, it was found that solidification heat was generated and freezing started. In addition, it was also possible to confirm that ice formation actually started at the timing. Thereafter, the temperature was around 0°C while the certain time passes. Here the product was in a state where there was a mixture of water and ice. The temperature finally started to decrease again from 0°C. At this time, the solution portion became ice while being disappeared. Accordingly, the temperature being measured was the temperature of a solid body within the ice, that is, was not the liquid temperature.

Hereinafter, with respect to "A", "B" and "C", the temperature difference when the liquid temperature of the non-cooled surface became a melting point (0°C), the temperature difference immediately before the temperature of a shelf was decreased from -10°C to -20°C, and the temperature difference immediately before the generation of solidification heat were described. The "temperature difference immediately before" referred to in the present invention indicates the highest temperature within the temperature difference which could be detected between a period of 1 second before the event and 20 seconds before the event.

### "A"

The temperature difference when the liquid temperature of the non-cooled surface became a melting point(0°C): 1.1°C
The temperature difference immediately before the temperature was decreased from -10°C to -20°C: 0.2°C
The temperature difference immediately before the generation of solidification heat: 1.1°C

### "B"

The temperature difference when the liquid temperature of the non-cooled surface became a melting point (0°C): 1.0°C
The temperature difference immediately before the temperature was decreased from -10°C to -20°C: 0.1°C
The temperature difference immediately before the generation of solidification heat: 0.9°C

### "C"

The temperature difference when the liquid temperature of the non-cooled surface became a melting point (0°C): 1.8°C
The temperature difference immediately before the temperature was decreased from -10°C to -20°C: 1.1°C
The temperature difference immediately before the generation of solidification heat: 2.1°C

Hereinafter, these products are called "porous bodies in a freezing process with a small temperature difference".

In addition, with respect to "D", "E", and "F", it was found that a product was in a state in which the temperature difference was higher than or equal to 4°C and the temperature difference became large up to immediately before the generation of solidification heat in a state (unfrozen state·supercooled state) in which the liquid surface became lower than or equal to 0°C as a melting point by being rapidly cooled in a shelf at -40°C or -60°C shown from Figs. 4 to 6.

Hereinafter, with respect to "D", "E", and "F", the temperature difference when the liquid temperature of the non-cooled surface became a melting point (0°C) and the temperature difference immediately before the generation of solidification heat were described.

### "D"

The temperature difference when the liquid temperature of the non-cooled surface became a melting point(0°C): 5.4°C
The temperature difference immediately before the generation of solidification heat: 4.2°C

### "E"

The temperature difference when the liquid temperature of the non-cooled surface became a melting point(0°C): 6.9°C
The temperature difference immediately before the generation of solidification heat: 5.3°C

### "F"

The temperature difference when the liquid temperature of the non-cooled surface became a melting point(0°C): 5.9°C
The temperature difference immediately before the generation of solidification heat: 3.3°C

Hereinafter, these products are called "porous bodies in a freezing process with a large temperature difference".

### [Example 3]

### [Freezing Process with Small Temperature Difference and Drying Process, in 1 Mass% Ethanol-Containing Solution]

A 1% (w/w) ethanol-containing aqueous CBE3 solution was made to flow into a PTFE thickness·cylindrical container and an aluminum glass·cylindrical container and was cooled down from the bottom surface within a vacuum freeze dryer (TF5-85ATNNN: Takara Co., Ltd.) using a cooling shelf. The melting point became -0.4°C by setting the solution as an ethanol-containing aqueous solution with a final concentration of 1 mass%. The change in the melting point in a ratio of the ethanol to the concentration of water was calculated while referring to literature "Pickering S.U.: A Study of the Properties of some Strong Solutions. J.Chem.Soc.London 63 (1893) 998-1027".

A combination of settings for the final concentration of the container and the aqueous CBE3 solution, the liquid amount, and the temperature of the shelf at this time was prepared as described below.
"AA" Final concentration of PTFE thickness·cylindrical container and aqueous CBE3 solution being 4 mass%, final concentration of ethanol being 1 mass%, and amount of aqueous solution being 4 mL. As the setting of the temperature of a shelf, the temperature was cooled down until the temperature becomes -10°C, and then, freezing was performed for 1 hour at -10°C, for 2 hours at -20°C, further for 3 hours at -40°C, and finally for 1 hour at -50°C. Thereafter, this frozen product was subjected to vacuum drying for 24 hours at -20°C after the setting of the temperature of the shelf was returned to -20°C. After 24 hours, the temperature of the shelf was increased to 20°C in a state in which the vacuum drying was continued as it was, and the vacuum drying was further performed for 48 hours at 20°C until the vacuum degree was sufficiently decreased. Then, the product was taken out of the vacuum freeze dryer. Accordingly, a porous body was obtained.
"BB" Final concentration of aluminum·glass·cylindrical container and aqueous CBE3 solution being 4 mass%, final concentration of ethanol being 1 mass%, and amount of aqueous solution being 4 mL. As the setting of the temperature of a shelf, the temperature was cooled down until the temperature becomes -10°C, and then, freezing was performed for 1 hour at -10°C, for 2 hours at -20°C, further for 3 hours at -40°C, and finally for 1 hour at -50°C. Thereafter, this frozen product was subjected to vacuum drying for 24 hours at -20°C after the setting of the temperature of the shelf was returned to -20°C. After 24 hours, the temperature of the shelf was increased to 20°C in a state in which the vacuum drying was continued as it was, and the vacuum drying was further performed for 48 hours at 20°C until the vacuum degree was sufficiently decreased. Then, the product was taken out of the vacuum freeze dryer. Accordingly, a porous body was obtained.

### [Measurement of Temperature Difference of 1 Mass% Ethanol-Containing Solution in Freezing Process]

With respect to each of "AA" and "BB", the liquid temperature of the surface of water in the central portion of a circle within a container was measured as a liquid temperature (a liquid temperature of the non-cooled surface) of a place which is farthest from a cooling side within a solution and the liquid temperature of the bottom portion within the container was measured as a liquid temperature (liquid temperature of the cooled surface) of a place which is closest to the cooling side within the solution. Here, the melting point of a solvent becomes -0.4°C since 1 mass% ethanol becomes the solvent. The change in the melting point in a ratio of the ethanol to the concentration of water was calculated while referring to literature "Pickering S.U.: A Study of the Properties of some Strong Solutions. J.Chem.Soc.London 63 (1893) 998-1027".

As a result, each temperature and each profile of the temperature difference thereof were as in each of Figs. 7 and 8.

In "AA" and "BB" from Figs. 7 and 8, it was found that the liquid temperature in a section, in which the temperature of a shelf was set to -10°C, was below -0.4°C as a melting point, and in the state, a product was in an (unfrozen·supercooled) state in which freezing did not occur. In addition, in this state, the temperature difference between the liquid temperature of the cooled surface and the liquid temperature of the non-cooled surface became lower than or equal to 2°C. Thereafter, a timing, at which the liquid temperature is rapidly increased to around -0.4°C by further decreasing the temperature of the shelf to -20°C, was confirmed. Here, it was found that solidification heat was generated and freezing started. In addition, it was also possible to confirm that ice formation actually started at the timing. Thereafter, the temperature was around 0.4°C while the certain time passes. Here, the product was in a state where there was a mixture of water and ice. The temperature finally started to decrease again from 0°C. At this time, the liquid portion became ice while being disappeared. Accordingly, the temperature being measured was the temperature of a solid body within the ice, that is, was not the liquid temperature.

Hereinafter, with respect to "AA" and "BB", the temperature difference when the liquid temperature of the non-cooled surface became a melting point (-0.4°C), the temperature difference immediately before the temperature of a shelf was decreased from -10°C to -20°C, and the temperature difference immediately before the generation of solidification heat were described.

### "AA"

The temperature difference when the liquid temperature of the non-cooled surface became a melting point(-0.4°C): 0.8°C
The temperature difference immediately before the temperature was decreased from -10°C to -20°C: 0.3°C
The temperature difference immediately before the generation of solidification heat: 0.8°C

### "BB"

The temperature difference when the liquid temperature of the non-cooled surface became a melting point(-0.4°C): 1.3°C
The temperature difference immediately before the temperature was decreased from -10°C to -20°C: 0.0°C
The temperature difference immediately before the generation of solidification heat: 1.3°C

As a result, similarly to "A" or "B", the products could be produced as "porous bodies in a freezing process with a small temperature difference".

### [Example 4]

### [Evaluation of Evenness of Structure of Porous Bodies]

An evaluation of evenness of a porous structure was performed on CBE3 porous bodies obtained in "A", "B", "C", "D", "E", "F", "AA", and "BB". Each of the obtained porous bodies was subjected to thermal cross-linking for 20 hours at 160°C to be made to be insoluble, and was then swollen in a normal saline solution for a sufficient time period. Thereafter, a frozen tissue piece was produced using a microtome, and a Hematoxylin-Eosin (HE) dye sample was prepared. The obtained sample images are shown in Figs. 9 to 12. A two-dimensional Fourier transformation image shown in Figs. 9 to 12 was obtained by performing two-dimensional Fourier transformation (FFT) on 1.5 mm square field of view in the obtained sample image using image analysis software ImageJ. In a case where uneven directional properties are confirmed in the preparation due to the two-dimensional Fourier transformation, a power spectrum which is particularly strong in the axial direction is observed. This technique of evaluating the unevenness is a technique which is obtained by applying a well-known technique in non-patent literature "Proceedings of the Japan Concrete Institute, vol. 22, No. 1, 2000, p. 211, Basic Study on Visual Assessment for Concrete Stain with 2 Dimension Fourier Transform, Manabu KANEMATSU, Ryoma KITAGAKI, Takafumi NOGUCHI, and Fuminori TOMOSAWA" or the like, to the evaluation of evenness of a porous body.

Furthermore, in the present invention, the presence and absence of the power spectrum in the axial direction was regulated by taking a line profile with respect to a lower end of an y-axis of a two-dimensional Fourier transformation image. Specifically, a graph, in which brightness values in the vicinity of an x-axis coordinate with respect to one tenth pixel width of the pixel size of the image from the lower end of the y-axis were plotted by being averaged, was created. For example, the line profile was measured as a line profile with 51-pixel width from the two-dimensional Fourier transformation image of 512 pixels x 512 pixels using software "Gwyddion 2.31". Each line profile is shown in Figs. 9 to 12. Accordingly, when a variation value of a base of an obtained line profile was set to σ, in a case where a peak of greater than or equal to 2.0 σ was detected in a central portion (at a region where x on the line profile was half of the maximum value), it was determined that there was a power spectrum in the axial direction. In this case where there was a power spectrum, the porous body was regarded as an uneven porous body. In a case where there was no power spectrum, the porous body was regarded as an even porous body.

Accordingly, it was found that "A", "B", "C", "AA", and "BB" which are porous bodies in a freezing process with a small temperature difference are even porous bodies. As shown in Examples 2 and 3, temperature differences immediately before the generation of solidification heat in the process of producing these porous bodies were respectively 1.1°C, 0.9°C, 2.1°C, 0.8°C, and 1.3°C.

In contrast, it was found that "D" (2.0 σ), "E" (6.0), and "F" (3.5 σ) which were porous bodies in a freezing process with a large temperature difference were uneven porous bodies. As shown in Example 2, the temperature differences immediately before the generation of solidification heat in the process of producing these porous bodies were respectively 4.2°C, 5.3°C, and 3.3°C.

Accordingly, it was found that, in the supercooled·unfrozen state in which the temperature is lower than or equal to a melting point of a solvent (0°C in a case of water and -0.4°C in a case of 1 mass% ethanol aqueous solution), the process included a process in which the difference between the liquid temperature (liquid temperature of the non-cooled surface) of a place which is farthest from a cooling side within a solution and the liquid temperature (liquid temperature of the cooled surface) of a place which is closest to the cooling side within the solution becomes within 2.5°C, and that even porous bodies can be obtained by making the temperature differences immediately before the generation of solidification heat be within 2.5°C.

### [Example 5] Production (Grinding and Cross-linking of Porous bodies) of Block with Small Temperature Difference from Porous Bodies in Freezing Process with Small Temperature Difference

The CBE3 porous bodies of "A" and "B" obtained in Example 1 (Example 2 for the measurement of temperature difference) were ground using new PowerMILL (Osaka Chemical Co., Ltd., new PowerMILL PM-2005). The grinding was performed for one minute x 5 times, that is, for 5 minutes in total at the maximum number of revolutions. The sizes of the obtained ground substances were divided using a stainless steel sieve to obtain uncrosslinked blocks at 25 µm to 53 µm, 53 µm to 106 µm, and 106 µm to 180 µm. Thereafter, CBE3 sample blocks were obtained by performing thermal cross-linking (performed in 6 levels of cross-linking times; 8 hours, 16 hours, 24 hours, 48 hours, 72 hours, and 96 hours) at 160°C under reduced pressure. Hereinafter, a block derived from a porous body of "A" on which cross-linking was performed for 48 hours was called E and a block derived from a porous body of "B" on which cross-linking was performed for 48 hours was called F. That is, E and F were blocks with a small temperature difference which were produced from the porous bodies in a freezing process with a small temperature difference. In the evaluation of this application, there was no influence of the difference in the cross-linking time on the performance. Therefore, here, porous bodies cross-linked for 48 hours were representatively used.

### [Comparative Example 2] Production (Grinding and Cross-linking of Porous bodies) of Block with Large Temperature Difference from Porous Bodies in Freezing Process with Large Temperature Difference

The CBE3 porous bodies of "D", "E", and "F" obtained in Comparative Example 1 (Example 2 for the measurement of temperature difference) were ground using new PowerMILL (Osaka Chemical Co., Ltd., new PowerMILL PM-2005). The grinding was performed for one minute x 5 times, that is, for 5 minutes in total at the maximum number of revolutions. The sizes of the obtained ground substances were divided using a stainless steel sieve to obtain uncrosslinked blocks at 25 µm to 53 µm, 53 µm to 106 µm, and 106 µm to 180 µm. Thereafter, CBE3 sample blocks were obtained by performing thermal cross-linking (performed for 24 hours and 48 hours of the cross-linking time) at 160°C under reduced pressure. Hereinafter, a block derived from a porous body of "D" on which cross-linking was performed for 48 hours was called A, a block derived from a porous body of "E" on which cross-linking was performed for 48 hours was called B, and, a block derived from a porous body of "F" on which cross-linking was performed for 48 hours was called C. That is, A, B, and C were blocks with a large temperature difference which were produced from the porous bodies in a freezing process with a large temperature difference. In the evaluation of this application, there was no influence of the difference in the cross-linking time on the performance. Therefore, here, porous bodies cross-linked for 48 hours were representatively used.

### [Example 6] Production of Mosaic Cell Aggregation using Block with Small Temperature Difference (hMSC)

Human bone marrow-derived mesenchymal stem cells (hMSC) were adjusted to be 100000 cells/mL using a proliferation medium (TAKARA BIO INC.: MSCGM BulletKit™), and a CBE3 block at 53 µm to 106 µm which had been produced in Example 5 was added thereto so as to make a concentration of 0.1 mg/mL. Then, 200 µL of the mixture was sown in a Sumilon Celltight X96U plate (of which the bottom is in a U shape, Sumitomo Bakelite Co., Ltd.), the plate was centrifuged in Mini Plate Centrifuge (600 g, 5 minutes) and was allowed to stand for 24 hours, to produce a mosaic cell aggregation, which was formed of the CBE3 block and hMSC cells and was in a spherical shape with a diameter of 1 mm (0.001 µg of a block per cell). Since this mosaic cell aggregation was produced in a U-shaped plate, the mosaic cell aggregation was in a spherical shape. In addition, it was possible to produce a mosaic cell aggregation similarly to the above even in any blocks of E and F.

### [Comparative Example 3] Production of Mosaic Cell Aggregation using Block with Large Temperature Difference (hMSC)

Human bone marrow-derived mesenchymal stem cells (hMSC) were adjusted to be 100000 cells/mL using a proliferation medium (TAKARA BIO INC.: MSCGM BulletKit™), and a CBE3 block at 53 µm to 106 µm which had been produced in Comparative Example 2 was added thereto so as to make a concentration of 0.1 mg/mL. Then, 200 µL of the mixture was sown in a Sumilon Celltight X96U plate (of which the bottom is in a U shape, Sumitomo Bakelite Co., Ltd.), the plate was centrifuged in Mini Plate Centrifuge (600 g, 5 minutes) and was allowed to stand for 24 hours, to produce a mosaic cell aggregation, which was formed of the CBE3 block and hMSC cells and was in a spherical shape with a diameter of 1 mm (0.001 µg of a block per cell). Since this mosaic cell aggregation was produced in a U-shaped plate, the mosaic cell aggregation was in a spherical shape. In addition, it was possible to produce a mosaic cell aggregation similarly to the above even in any blocks of A, B, and C.

### [Example 7] Production of Mosaic Cell Aggregation using Block with Small Temperature Difference (hMSC + hECFC)

Human vascular endothelial precursor cells (hECFC) were adjusted to be 100000 cells/mL using a proliferation medium (LONZA: EGM-2+ECFC serum supplement), and a CBE3 block at 53 µm to 106 µm which had been produced in Example 5 was added thereto so as to make a concentration of 0.05 mg/mL. Then, 200 µL of the mixture was sown in a Sumilon Celltight X96U plate (of which the bottom is in a U shape, Sumitomo Bakelite Co., Ltd.), the plate was centrifuged in Mini Plate Centrifuge (600 g, 5 minutes) and was allowed to stand for 24 hours, to produce a mosaic cell aggregation, which was formed of ECFC and the CBE3 block and was in a flat shape, was produced. Then, the medium was removed, human bone marrow-derived mesenchymal stem cells (hMSC) were adjusted to be 100000 cells/mL using a proliferation medium (TAKARA BIO INC.: MSCGM BulletKit™), and a CBE3 block at 53 µm to 106 µm which had been produced in Example 5 was added thereto so as to make a concentration of 0.1 mg/mL. Then, 200 µL of the mixture in which an hECFC mosaic cell aggregation was contained was sown in a Sumilon Celltight X96U plate, the plate was centrifuged in Mini Plate Centrifuge (600 g, 5 minutes) and was allowed to stand for 24 hours, to produce a mosaic cell aggregation, which was formed of hMSC, hECFC, and the CBE3 block and was in a spherical shape with a diameter of 1 mm. In addition, it was possible to produce a mosaic cell aggregation similarly to the above even in any blocks of E and F.

### [Comparative Example 4] Production of Mosaic Cell Aggregation using Block with Large Temperature Difference (hMSC + hECFC)

Human vascular endothelial precursor cells (hECFC) were adjusted to be 100000 cells/mL using a proliferation medium (LONZA: EGM-2+ECFC serum supplement), and a CBE3 block at 53 µm to 106 µm which had been produced in Comparative Example 2 was added thereto so as to make a concentration of 0.05 mg/mL. Then, 200 µL of the mixture was sown in a Sumilon Celltight X96U plate, the plate was centrifuged in Mini Plate Centrifuge (600 g, 5 minutes) and was allowed to stand for 24 hours, to produce a mosaic cell aggregation, which was formed of ECFC and the CBE3 block and was in a flat shape, was produced. Then, the medium was removed, human bone marrow-derived mesenchymal stem cells (hMSC) were adjusted to be 100000 cells/mL using a proliferation medium (TAKARA BIO INC.: MSCGM BulletKit™), and a CBE3 block at 53 µm to 106 µm which had been produced in Comparative Example 2 was added thereto so as to make a concentration of 0.1 mg/mL. Then, 200 µL of the mixture in which an hECFC mosaic cell aggregation was contained was sown in a Sumilon Celltight X96U plate, the plate was centrifuged in Mini Plate Centrifuge (600 g, 5 minutes) and was allowed to stand for 24 hours, to produce a mosaic cell aggregation, which was formed of hMSC, hECFC, and the CBE3 block and was in a spherical shape with a diameter of 1 mm. In addition, it was possible to produce a mosaic cell aggregation similarly to the above even in any blocks of A, B, and C.

### [Example 8] Merging of Mosaic Cell Aggregations (hMSC) using Block with Small Temperature Difference

5 mosaic cell aggregations (using CBE3 blocks of the present invention) on day 2 which had been produced in Example 6 were arranged in a Sumilon Celltight X96U plate, and culturing was performed for 24 hours. As a result, it became clear that mosaic cell aggregations were naturally merged by bonding cells, which were disposed at outer peripheral portions between the mosaic cell aggregations, to each other. In addition, it was possible to produce mosaic cell aggregations similarly to the above even in any blocks of E and F.

### [Example 9] Merging of Mosaic Cell Aggregations (hMSC + hECFC) using Block with Small Temperature Difference

5 mosaic cell aggregations (using a CBE3 block of the present invention) on day 2 which had been produced in Example 7 were arranged in a Sumilon Celltight X96U plate, and culturing was performed for 24 hours. As a result, it became clear that mosaic cell aggregations were naturally merged by bonding cells, which were disposed at outer peripheral portions between the mosaic cell aggregations, to each other. In addition, it was possible to produce mosaic cell aggregations similarly to the above even in any blocks of E and F.

### [Comparative Example 5] Merging of Mosaic Cell Aggregations (hMSC) using Block with Large Temperature Difference

5 mosaic cell aggregations (derived from CBE3 blocks for comparison) on day 2 which had been produced in Comparative Example 3 were arranged in a Sumilon Celltight X96U plate, and culturing was performed for 24 hours. As a result, it became clear that mosaic cell aggregations were naturally merged by bonding cells, which were disposed at outer peripheral portions between the mosaic cell aggregations, to each other. In addition, it was possible to produce mosaic cell aggregations similarly to the above even in any blocks of A, B, and C.

### [Comparative Example 6] Merging of Mosaic Cell Aggregations (hMSC + hECFC) using Block with Large Temperature Difference

5 mosaic cell aggregations (derived from CBE3 blocks for comparison) on day 2 which had been produced in Comparative Example 4 were arranged in a Sumilon Celltight X96U plate, and culturing was performed for 24 hours. As a result, it became clear that mosaic cell aggregations were naturally merged by bonding cells, which were disposed at outer peripheral portions between the mosaic cell aggregations, to each other. In addition, it was possible to produce mosaic cell aggregations similarly to the above even in any blocks of A, B, and C.

### [Example 10] Transplantation of Mosaic Cell Aggregations Using CBE3 Block

NOD/SCID male mice (Charles River Laboratories International, Inc.) at the ages of 4 weeks to 6 weeks were used. In the anesthetized condition, body hair of the abdomen of the mice was removed, a cut was made under the skin of the upper abdomen, scissors were inserted through the cut, and the skin was peeled off from the muscle. Then, the hMSC mosaic cell aggregations and the hMSC + hECFC mosaic cell aggregations which have been produced in Examples 8 and 9 and Comparative Examples 5 and 6 were picked up, and were transplanted under the skin which is near the lower abdomen and is 1.5 cm away from the cut, and the cut portion of the skin was sutured.

### [Example 11] Collecting Mosaic Cell Aggregation Using CBE3 Block

Dissecting was performed 2 weeks after the transplantation. The skin of the abdomen was peeled off and the skin to which the mosaic cell aggregations were adhered was cut off in a square shape with a size of about 1 square cm. In a case where the mosaic cell aggregations were adhered also in the muscle of the abdomen, the muscle was also collected together with the mosaic cell aggregations.

### [Example 12] Sample Analysis

A piece of the skin to which mosaic cell aggregations were adhered, and a tissue piece with respect to mosaic cell aggregations before transplantation were produced. The skin was immersed into 4 mass% paraformaldehyde, and formalin fixation was performed. Then, the skin was embedded by paraffin, and a tissue piece of the skin containing mosaic cell aggregations was produced. The piece was subjected to Hematoxylin-Eosin (HE) dyeing.

HE samples of the mosaic cell aggregations 2 weeks after the transplantation performed in Example 10 are shown in Figs. 13 to 17. With respect to a mosaic cell aggregation with patterns, three enlarged images are shown and the arrows indicate blood vessels. There are six hMSC mosaic cell aggregations of A and B, and among these, three hMSC mosaic cell aggregations are shown. The number of cells is the number of overall survival cells in an image, and the number of blood vessels is a value in which the number of blood vessels in each image is converted per the area. The CV value is a proportion at the time when the standard deviation of three or six mosaic cell aggregations using blocks is divided by an average value, and a higher numerical value shows that the variations between individuals become larger. In addition, the summary of these results is shown in the following Tables 1 to 4.

**[Table 1]**

| hMSC mosaic cell aggregation | | | |
|---|---|---|---|
| | | Average number of cells (cells) | CV value (%) between individuals |
| Large temperature difference | A | 122 | 59 |
| | B | 119 | 31 |
| | C | 122 | 32 |
| Small temperature difference | E | 256 | 7 |
| | F | 194 | 21 |

**[Table 2]**

| hMSC mosaic cell aggregation | | | |
|---|---|---|---|
| | | Average number of blood vessels (piece/mm2) | CV value (%) between individuals |
| Large temperature difference | A | 12 | 137 |
| | B | 10 | 155 |
| | C | 17 | 125 |
| Small temperature difference | E | 58 | 10 |
| | F | 54 | 89 |

**[Table 3]**

| hMSC + hECFC mosaic cell aggregation | | | |
|---|---|---|---|
| | | Average number of cells (cells) | CV value (%) between individuals |
| Large temperature difference | A | 84 | 70 |
| | B | 158 | 39 |
| Small temperature difference | E | 202 | 29 |
| | F | 171 | 29 |

**[Table 4]**

| hMSC + hECFC mosaic cell aggregation | | | |
|---|---|---|---|
| | | Average number of blood vessels (piece/mm2) | CV value (%) between individuals |
| Large temperature difference | A | 34 | 173 |
| | B | 51 | 87 |
| Small temperature difference | E | 122 | 17 |
| | F | 109 | 29 |

The results in the hMSC mosaic cell aggregations used in respective blocks are as follows.

In Fig. 13, the results in the hMSC mosaic cell aggregations using blocks A and B with a large temperature difference are shown. In the block A with a large temperature difference, the average number of cells is 122 cells and the average number of blood vessels is 12 pieces/mm², and the respective CV values are 59% and 137%. In the block B with a large temperature difference, the average number of cells is 119 cells and the average number of blood vessels is 10 pieces/mm², and the respective CV values are 31% and 155%. It can be seen from the piece images that there are also variations in the mosaic cell aggregations.

In Fig. 14, the results in the hMSC mosaic cell aggregations using a block C with a large temperature difference are shown. In the block C with a large temperature difference, the average number of cells is 122 cells and the average number of blood vessels is 17 pieces/mm², and the respective CV values are 32% and 125%. Similarly, it can be seen from the piece images that there are also variations in the mosaic cell aggregations.

In Fig. 15, the results in the hMSC mosaic cell aggregations using blocks E and F with a small temperature difference are shown. In the block E with a large temperature difference, the average number of cells is 256 cells and the average number of blood vessels is 58 pieces/mm², and the respective CV values are 7% and 10%. In the block F with a large temperature difference, the average number of cells is 194 cells and the average number of blood vessels is 54 pieces/mm², and the respective CV values are 21% and 89%. It can be seen from the piece images that there are also less variations in the mosaic cell aggregations.

The results in the hMSC + hECFC mosaic cell aggregations used in blocks are as follows.

In Fig. 16, the results in the hMSC + hECFC mosaic cell aggregations using blocks A and B with a large temperature difference are shown. In the block A with a large temperature difference, the average number of cells is 84 cells and the average number of blood vessels is 34 pieces/mm², and the respective CV values are 70% and 173%. In the block B with a large temperature difference, the average number of cells is 158 cells and the average number of blood vessels is 51 pieces/mm², and the respective CV values are 39% and 87%. It can be seen from the piece images that there are also variations in the mosaic cell aggregations.

In Fig. 17, the results in the hMSC + hECFC mosaic cell aggregations using blocks E and F with a small temperature difference are shown. In the block E with a large temperature difference, the average number of cells is 202 cells and the average number of blood vessels is 122 pieces/mm², and the respective CV values are 29% and 17%. In the block F with a large temperature difference, the average number of cells is 171 cells and the average number of blood vessels is 109 pieces/mm², and the respective CV values are 29% and 29%. It can be seen from the piece images that there are also no variations in the mosaic cell aggregations.

Generally, the results are as summarized in Tables 1 to 4. It can be seen that, in a case of using a block with a large temperature difference, the individual difference between the mosaic cell aggregations is large and varies. Even regarding the average number of cells and the average number of blood vessels, it can also be seen that there is no significant difference caused by the kinds of blocks with a large temperature difference.

In contrast, it can be seen that, in a case of using a block with a small temperature difference, the individual difference between the mosaic cell aggregations is small and there are less variations. The average number of cells and the average number of blood vessels in the block with a small temperature difference also show significantly higher value than those in the block with a large temperature difference, and therefore, it can be said that blood vessels are easily formed and many cells can be made to survive. It is considered that this is because the structure of the porous bodies obtained in the freezing process with a small temperature difference as shown in Example 4 is even, and therefore, the variations in the performance of the block with a small temperature difference produced from the porous bodies thereof within a living body are less. Furthermore, unexpectedly, it was found that such a point also contributes to an ability of forming blood vessels and viability of cells. In addition, it is significantly important to improve the ability of forming blood vessels and the viability of transplantation cells in order to exhibit the performance of transplantation cells. The porous bodies of the present invention exhibit a high effect from the viewpoint of improving the two aspects.

### [Example 13] Calculation of Proportion and Concentration of ECFC in hMSC + hECFC Mosaic Cell Aggregation

In the mosaic cell aggregation representatively using E in Example 7, a piece was subjected to immunological dyeing using kits (for Universal K0673 of a Dako LSAB2 kit and for a dual rabbit·mouse primary antibody of Dako LSAB2 kit/HRP (DAB)) in which DAB color development in a CD31 antibody (EPT Anti CD31/PECAM-1) for hECFC staining. The proportion of the area of hECFC (vascular cell) in the central portion was obtained using a dyeing method utilizing image treatment software ImageJ and the CD31 antibody. The "central portion" referred to herein is defined above.

As a result, the proportion of the area of hECFC (vascular cell) in the central portion of the mosaic cell aggregation representatively using E in Example 7 was 98%. Furthermore, regarding the mosaic cell aggregation representatively using E in Example 7, the density of cells of hECFC existing in the central portion was calculated by overlapping the dye using the above-described CD31 antibody and the Hematoxylin-Eosin (HE) dye each other. The density of vascular cells in the central portion can be obtained by actually counting the number of cells of a thin slice sample and dividing the number of cells by the volume. First, the number of cells was calculated after overlapping the above-described two sheets of images using Photoshop, and counting the number of cell nuclei of the HE stain which overlapped the dye using the above-described CD31 antibody. In contrast, the volume was obtained after obtaining the area of the central portion using ImageJ and multiplying the obtained area by 2 µm of the thickness of the thin slice sample thereof. As a result, the number of cells of hECFC (vascular cells) in the central portion of the mosaic cell aggregation representatively using E in Example 7 was 2.3 x 10⁻⁴ cells/µm³.

## Claims

1. A method for producing a biocompatible macromolecular porous body, comprising:
a step (a) of cooling a solution of biocompatible macromolecules to be in an unfrozen state, the difference between a temperature of a portion at the highest liquid temperature within the solution and a temperature of a portion at the lowest liquid temperature within the solution being lower than or equal to 2.5°C and the temperature of the portion at the highest liquid temperature within the solution being lower than or equal to a melting point of a solvent;
a step (b) of freezing the solution of biocompatible macromolecules obtained in the step (a); and
a step (c) of freeze-drying the frozen biocompatible macromolecules obtained in the step (b),
wherein the biocompatible macromolecules are gelatin, collagen, elastin, fibronectin, laminin, tenascin, fibrin, fibroin, entactin, thrombospondin, polylactic acid, polyglycolic acid, lactic acid-glycolic acid copolymers, hyaluronic acid, glycosaminoglycans, proteoglycans, chondroitin, cellulose, agarose, carboxymethyl cellulose, chitin, or chitosan.

2. The method for producing a biocompatible macromolecular porous body according to claim 1,
wherein, in the step (a), the difference between a temperature of a portion at the highest liquid temperature within the solution and a temperature of a portion at the lowest liquid temperature within the solution immediately before generation of solidification heat is lower than or equal to 2.5°C.

3. The method for producing a biocompatible macromolecular porous body according to claim 1 or 2,
wherein, in the step (a), the temperature of the portion at the lowest liquid temperature within the solution is lower than or equal to a melting point of the solvent -5°C.

4. The method for producing a biocompatible macromolecular porous body according to any one of claims 1 to 3,
wherein the biocompatible macromolecules are recombinant gelatin.

5. The method for producing biocompatible macromolecular porous body according to claim 4,
wherein the recombinant gelatin is any of
(a) protein having an amino acid sequence described in SEQ ID No: 1;
(b) protein which has an amino acid sequence in which one or a plurality of amino acids are deleted, substituted, or added in the amino acid sequence described in SEQ ID No: 1, and has biocompatibility; or
(c) protein which has an amino acid sequence having 80% or higher homology to the amino acid sequence described in SEQ ID No: 1, and has biocompatibility.

6. A biocompatible macromolecular porous body,
wherein, with respect to a two-dimensional Fourier transformation image which is obtained by performing two-dimensional Fourier transformation on 1.5 mm square field of view of an image of a cross-sectional structure of the biocompatible macromolecular porous body, in a case where a line profile, in which brightness values in the vicinity of an x-axis coordinate with respect to one tenth pixel width of the pixel size of the image from a lower end of an y-axis of the two-dimensional Fourier transformation image are plotted on the y-axis by being averaged, is created, there is no peak at a region where x on the line profile is half of the maximum value,
wherein the biocompatible macromolecules are gelatin, collagen, elastin, fibronectin, laminin, tenascin, fibrin, fibroin, entactin, thrombospondin, -polylactic acid, polyglycolic acid, lactic acid-glycolic acid copolymers, hyaluronic acid, glycosaminoglycans, proteoglycans, chondroitin, cellulose, agarose, carboxymethyl cellulose, chitin, or chitosan.

7. The biocompatible macromolecular porous body according to claim 6,
wherein, in a case where a variation value of a base of the line profile is set to σ, a case, in which a peak of greater than or equal to 2.0 σ is not detected at a region where x on the line profile of the two-dimensional Fourier transformation image is half of the maximum value, is regarded that there is no peak.

8. The biocompatible macromolecular porous body according to claim 6 or 7, which is produced through the method for producing a biocompatible macromolecular porous body according to any one of claims 1 to 6.

9. The biocompatible macromolecular porous body according to any one of claims 6 to 8,
wherein the biocompatible macromolecules are recombinant gelatin.

10. The biocompatible macromolecular porous body according to claim 9,
wherein the recombinant gelatin is any of
(a) protein having an amino acid sequence described in SEQ ID No: 1;
(b) protein which has an amino acid sequence in which one or a plurality of amino acids are deleted, substituted, or added in the amino acid sequence described in SEQ ID No: 1, and has biocompatibility; or
(c) protein which has an amino acid sequence having 80% or higher homology to the amino acid sequence described in SEQ ID No: 1, and has biocompatibility.

11. A biocompatible macromolecular block which is obtained by grinding the biocompatible macromolecular porous body according to any one of claims 6 to 10.

12. A cell structure, comprising:
the biocompatible macromolecular block according to claim 11; and
at least one kind of cell,
wherein a plurality of the biocompatible macromolecular blocks are arranged in a gap between a plurality of cells.

## Patentansprüche

1. Verfahren zum Herstellen eines biokompatiblen makromolekularen porösen Körpers, umfassend:
einen Schritt (a) des Kühlens einer Lösung von biokompatiblen Makromolekülen, um in einem nichteingefrorenem Zustand zu sein, wobei der Unterschied zwischen einer Temperatur eines Teils mit der höchsten Flüssigkeitstemperatur innerhalb der Lösung und einer Temperatur eines Teils mit der niedrigsten Flüssigkeitstemperatur innerhalb der Lösung kleiner als oder gleich 2,5°C ist, und wobei die Temperatur des Teils mit der höchsten Flüssigkeitstemperatur innerhalb der Lösung niedriger als ein oder gleich einem Schmelzpunkt eines Lösungsmittels ist;
einen Schritt (b) des Einfrierens der in Schritt (a) erhaltenen Lösung von biokompatiblen Makromolekülen; und
einen Schritt (c) des Gefriertrocknens der in Schritt (b) erhaltenen eingefrorenen biokompatiblen Makromoleküle,
wobei die biokompatiblen Makromoleküle Gelatine, Kollagen, Elastin, Fibronektin, Laminin, Tenascin, Fibrin, Fibroin, Entactin, Thrombospondin, Polymilchsäure, Polyglykolsäure, Milchsäure-Glykolsäure-Copolymere, Hyaluronsäure, Glykosaminoglykane, Proteoglykane, Chondroitin, Cellulose, Agarose, Carboxymethylcellulose, Chitin oder Chitosan sind.

2. Verfahren zum Herstellen eines biokompatiblen makromolekularen porösen Körpers gemäß Anspruch 1,
wobei in dem Schritt (a) der Unterschied zwischen einer Temperatur eines Teils mit der höchsten Flüssigkeitstemperatur innerhalb der Lösung und einer Temperatur eines Teils mit der niedrigsten Flüssigkeitstemperatur innerhalb der Lösung unmittelbar vor Erzeugung von Erstarrungswärme kleiner als oder gleich 2,5°C ist.

3. Verfahren zum Herstellen eines biokompatiblen makromolekularen porösen Körpers gemäß Anspruch 1 oder 2,
wobei in dem Schritt (a) die Temperatur des Teils mit der niedrigsten Flüssigkeitstemperatur innerhalb der Lösung niedriger als ein oder gleich einem Schmelzpunkt des Lösungsmittels -5°C ist.

4. Verfahren zum Herstellen eines biokompatiblen makromolekularen porösen Körpers gemäß irgendeinem der Ansprüche 1 bis 3,
wobei die biokompatiblen Makromoleküle rekombinante Gelatine sind.

5. Verfahren zum Herstellen eines biokompatiblen makromolekularen porösen Körpers gemäß Anspruch 4,
wobei die rekombinante Gelatine irgendeines der folgenden ist:
(a) Protein mit einer Aminosäuresequenz, wie in SEQ ID No: 1 beschrieben;
(b) Protein, welches eine Aminosäuresequenz hat, in welcher eine oder eine Mehrzahl von Aminosäuren in der Aminosäuresequenz, die in SEQ ID No: 1 beschrieben ist, deletiert, substituiert oder hinzugefügt sind, und welches Biokompatibilität hat; oder
(c) Protein, welches eine Aminosäuresequenz mit 80% oder höherer Homologie zu der Aminosäuresequenz hat, die in SEQ ID No: 1 beschrieben ist, und welches Biokompatibilität hat.

6. Biokompatibler makromolekularer poröser Körper,
wobei in Bezug auf ein zweidimensionales Fourier-Transformationsbild, welches durch das Durchführen zweidimensionaler Fourier-Transformation auf 1,5 mm quadratischem Sichtfeld eines Bilds einer Querschnittsstruktur des biokompatiblen makromolekularen porösen Körpers erhalten wird, in einem Fall, in dem ein Linienprofil erzeugt wird, in welchem Helligkeitswerte in der Nähe einer x-Achsenkoordinate bezüglich einer Zehntel-Pixelbreite der Pixelgröße des Bilds vom unteren Ende einer γ-Achse des zwei-dimensionalen Fourier-Transformationsbilds durch Mitteln auf der γ-Achse graphisch dargestellt werden, es bei einer Region, in der x auf dem Linienprofil die Hälfte des Maximalwerts beträgt, keinen Peak gibt,
wobei die biokompatiblen Makromoleküle Gelatine, Kollagen, Elastin, Fibronektin, Laminin, Tenascin, Fibrin, Fibroin, Entactin, Thrombospondin, Polymilchsäure, Polyglykolsäure, Milchsäure-Glykolsäure-Copolymere, Hyaluronsäure, Glykosaminoglykane, Proteoglykane, Chondroitin, Cellulose, Agarose, Carboxymethylcellulose, Chitin oder Chitosan sind.

7. Biokompatibler makromolekularer poröser Körper gemäß Anspruch 6,
wobei, in einem Fall, in dem ein Variationswert einer Basis des Linienprofils als σ gesetzt wird, einem Fall, in dem ein Peak von mehr als oder gleich 2,0 σ in einer Region nicht detektiert wird, in der x auf dem Linienprofil des zweidimensionalen Fourier-Transformationsbilds die Hälfte des Maximalwerts beträgt, dies als kein Peak angesehen wird.

8. Biokompatibler makromolekularer poröser Körper gemäß Anspruch 6 oder 7, welcher durch das Verfahren zum Herstellen eines biokompatiblen makromolekularen porösen Körpers gemäß irgendeinem der Ansprüche 1 bis 6 hergestellt wird.

9. Biokompatibler makromolekularer poröser Körper gemäß irgendeinem der Ansprüche 6 bis 8,
wobei die biokompatiblen Makromoleküle rekombinante Gelatine sind.

10. Biokompatibler makromolekularer poröser Körper gemäß Anspruch 9,
wobei die rekombinante Gelatine irgendeines der folgenden ist:
(a) Protein mit einer Aminosäuresequenz, wie beschrieben in SEQ ID No: 1;
(b) Protein, welches eine Aminosäuresequenz hat, in welcher eine oder eine Mehrzahl von Aminosäuren in der Aminosäuresequenz, die in SEQ ID No: 1 beschrieben ist, deletiert, substituiert oder hinzugefügt sind, und welches Biokompatibilität hat; oder
(c) Protein, welches eine Aminosäuresequenz mit 80% oder höherer Homologie zu der Aminosäuresequenz hat, die in SEQ ID No: 1 beschrieben ist, und welches Biokompatibilität hat.

11. Biokompatibler makromolekularer Block, welcher durch Mahlen des biokompatiblen makromolekularen porösen Körpers gemäß irgendeinem der Ansprüche 6 bis 10 erhalten wird.

12. Zellstruktur, umfassend:
den biokompatiblen makromolekularen Block gemäß Anspruch 11; und
mindestens eine Art von Zelle,
wobei eine Mehrzahl der biokompatiblen makromolekularen Blöcke in einer Lücke zwischen einer Mehrzahl von Zellen angeordnet sind.

## Revendications

1. Procédé de production d'un corps poreux macromoléculaire biocompatible, comprenant :
une étape (a) de refroidissement d'une solution de macromolécules biocompatibles pour qu'elle soit dans un état non congelé, la différence entre une température d'une partie à la température de liquide la plus élevée dans la solution et une température d'une partie à la température de liquide la plus basse dans la solution étant inférieure ou égale à 2,5 °C et la température de la partie à la température de liquide la plus élevée dans la solution étant inférieure ou égale à un point de fusion d'un solvant ;
une étape (b) de congélation de la solution de macromolécules biocompatibles obtenue à l'étape (a) ; et
une étape (c) de lyophilisation des macromolécules biocompatibles congelées obtenues à l'étape (b),
dans lequel les macromolécules biocompatibles sont la gélatine, le collagène, l'élastine, la fibronectine, la laminine, la ténascine, la fibrine, la fibroïne, l'entactine, la thrombospondine, l'acide polylactique, l'acide polyglycolique, les copolymères d'acide lactique-acide glycolique, l'acide hyaluronique, les glycosaminoglycanes, les protéoglycanes, la chondroïtine, la cellulose, l'agarose, la carboxyméthylcellulose, la chitine, ou le chitosane.

2. Procédé de production d'un corps poreux macromoléculaire biocompatible selon la revendication 1,
dans lequel, à l'étape (a), la différence entre une température d'une partie à la température de liquide la plus élevée dans la solution et une température d'une partie à la température de liquide la plus basse dans la solution immédiatement avant la génération de chaleur de solidification est inférieure ou égale à 2,5 °C.

3. Procédé de production d'un corps poreux macromoléculaire biocompatible selon la revendication 1 ou 2,
dans lequel, à l'étape (a), la température de la partie à la température de liquide la plus basse dans la solution est inférieure ou égale à un point de fusion du solvant de -5°C.

4. Procédé de production d'un corps poreux macromoléculaire biocompatible selon l'une quelconque des revendications 1 à 3,
dans lequel les macromolécules biocompatibles sont de la gélatine recombinante.

5. Procédé de production d'un corps poreux macromoléculaire biocompatible selon la revendication 4,
dans lequel la gélatine recombinante est l'une quelconque de
(a) une protéine ayant une séquence d'acides aminés décrite dans SEQ ID NO : 1;
(b) une protéine qui a une séquence d'acides aminés dans laquelle un ou une pluralité d'acides aminés sont délétés, substitués, ou ajoutés dans la séquence d'acides aminés décrite dans SEQ ID NO : 1, et présente une biocompatibilité ; ou
(c) une protéine qui a une séquence d'acides aminés présentant une homologie de 80 % ou plus avec la séquence d'acides aminés décrite dans SEQ ID NO : 1, et présente une biocompatibilité.

6. Corps poreux macromoléculaire biocompatible,
dans lequel, par rapport à une image par transformée de Fourier en 2D qui est obtenue par réalisation d'une transformée de Fourier en 2D sur un champ de vision de 1,5 mm² d'une image d'une structure en coupe transversale du corps poreux macromoléculaire biocompatible, dans un cas où un profil linéaire, dans lequel des valeurs de luminosité à proximité d'une coordonnée d'un axe des X par rapport à une largeur de pixel d'un dixième de la taille de pixel de l'image à partir d'une extrémité inférieure d'un axe des y de l'image par transformée de Fourier en 2D sont tracées sur l'axe des y par calcul de leur moyenne, est créé, il n'y a pas de pic au niveau d'une région où x sur le profil linéaire est la moitié de la valeur maximale,
dans lequel les macromolécules biocompatibles sont la gélatine, le collagène, l'élastine, la fibronectine, la laminine, la ténascine, la fibrine, la fibroïne, l'entactine, la thrombospondine, l'acide polylactique, l'acide polyglycolique, les copolymères d'acide lactique-acide glycolique, l'acide hyaluronique, les glycosaminoglycanes, les protéoglycanes, la chondroïtine, la cellulose, l'agarose, la carboxyméthylcellulose, la chitine, ou le chitosane.

7. Corps poreux macromoléculaire biocompatible selon la revendication 6,
dans lequel dans un cas où une valeur de variation d'une base du profil linéaire est définie à σ, un cas, dans lequel un pic supérieur ou égal à 2,0 σ n'est pas détecté dans une région où x sur le profil linéaire de l'image par transformée de Fourier en 2D est la moitié de la valeur maximale, est considéré comme n'ayant pas de pic.

8. Corps poreux macromoléculaire biocompatible selon la revendication 6 ou 7, qui est produit par le procédé de production d'un corps poreux macromoléculaire biocompatible selon l'une quelconque des revendications 1 à 6.

9. Corps poreux macromoléculaire biocompatible selon l'une quelconque des revendications 6 à 8,
dans lequel les macromolécules biocompatibles sont de la gélatine recombinante.

10. Corps poreux macromoléculaire biocompatible selon la revendication 9,
dans lequel la gélatine recombinante est une quelconque parmi
(a) une protéine ayant une séquence d'acides aminés décrite dans SEQ ID NO : 1;
(b) une protéine qui a une séquence d'acides aminés dans laquelle un ou une pluralité d'acides aminés sont délétés, substitués, ou ajoutés dans la séquence d'acides aminés décrite dans SEQ ID NO : 1, et présente une biocompatibilité ; ou
(c) une protéine qui a une séquence d'acides aminés présentant une homologie de 80 % ou plus avec la séquence d'acides aminés décrite dans SEQ ID NO : 1, et présente une biocompatibilité.

11. Bloc macromoléculaire biocompatible qui est obtenu par meulage du corps poreux macromoléculaire biocompatible selon l'une quelconque des revendications 6 à 10.

12. Structure cellulaire, comprenant :
le bloc macromoléculaire biocompatible selon la revendication 11; et
au moins une sorte de cellule,
dans lequel une pluralité des blocs macromoléculaires biocompatibles sont agencés dans un espace entre une pluralité de cellules.
